# EUROPEAN PATENT APPLICATION

(11) **EP 2 062 589 A1**
(43) Date of publication of application: **27.05.2009**
(21) Application number: 07829298.4
(22) Date of filing: 05.10.2007
(51) Int. Cl.: A61K 38/00, A61P 7/02, C07K 14/745, C12N 15/00

(54) **THERAPEUTIC AND/OR AMELIORATING AGENT FOR DISSEMINATED INTRAVASCULAR COAGULATION**

(30) Priority: 06.10.2006 JP 2006274573
(71) Applicant: Asahi Kasei Pharma Corporation, Tokyo 101-8101 (JP)
(72) Inventor: AOKI, Yoshikazu, Tokyo 100-8440 (JP)
(74) Representative: Forstmeyer, Dietmar
(86) International application number: PCT/JP2007/069560
(87) International publication number: WO 2008/044631

(57) **Abstract**

It is an object of the present invention to provide a therapeutically effective agent for therapy and/or improvement of DIC, or a method for treating and/or improving DIC. The present invention provides an agent for therapy and/or improvement of disseminated intravascular coagulation comprising thrombomodulin as an active ingredient, which is administered to antithrombin-reduced patients having a low plasma antithrombin activity of less than 50%.

SEQUENCE LISTING
<110> Asahi Kasei Pharma Kabushiki Kaisha
<120> An agent for therapy and/or improvement of disseminated intravascul
ar coagulation
<130> F107098-WO
<160> 13
<210> 1
<211> 132
<212> PRT
<213> human
<400> 1
Met Leu Gly Val Leu Val Leu Gly Ala Leu Ala Leu Ala Gly Leu Gly
1               5                   10                  15
Phe Pro Asp Pro Cys Phe Arg Ala Asn Cys Glu Tyr Gln Cys Gln Pro
            20                  25                  30
Leu Asn Gln Thr Ser Tyr Leu Cys Val Cys Ala Glu Gly Phe Ala Pro
        35                  40                  45
Ile Pro His Glu Pro His Arg Cys Gln Met Phe Cys Asn Gln Thr Ala
    50                  55                  60
Cys Pro Ala Asp Cys Asp Pro Asn Thr Gln Ala Ser Cys Glu Cys Pro
65                  70                  75                  80
Glu Gly Tyr Ile Leu Asp Asp Gly Phe Ile Cys Thr Asp Ile Asp Glu
                85                  90                  95
Cys Glu Asn Gly Gly Phe Cys Ser Gly Val Cys His Asn Leu Pro Gly
            100                 105                 110
Thr Phe Glu Cys Ile Cys Gly Pro Asp Ser Ala Leu Val Arg His Ile
        115                 120                 125
Gly Thr Asp Cys
    130
<210> 2
<211> 396
<212> DNA
<213> human
<400> 2
atgcttgggg tcctggtcct tggcgcgctg gccctggccg gcctggggtt ccccgacccg 60
tgcttcagag ccaactgcga gtaccagtgc cagcccctga accaaactag ctacctctgc 120
gtctgcgccg agggcttcgc gcccattccc cacgagccgc acaggtgcca gatgttttgc 180
aaccagactg cctgtccagc cgactgcgac cccaacaccc aggctagctg tgagtgccct 240
gaaggctaca tcctggacga cggtttcatc tgcacggaca tcgacgagtg cgaaaacggc 300
ggcttctgct ccggggtgtg ccacaacctc cccggtacct tcgagtgcat ctgcgggccc 360
gactcggccc ttgtccgcca cattggcacc gactgt                           396
<210> 3
<211> 132
<212> PRT
<213> human
<400> 3
Met Leu Gly Val Leu Val Leu Gly Ala Leu Ala Leu Ala Gly Leu Gly
  1               5                  10                  15

Phe Pro Asp Pro Cys Phe Arg Ala Asn Cys Glu Tyr Gln Cys Gln Pro
            20                  25                  30
Leu Asn Gln Thr Ser Tyr Leu Cys Val Cys Ala Glu Gly Phe Ala Pro
        35                  40                  45
Ile Pro His Glu Pro His Arg Cys Gln Met Phe Cys Asn Gln Thr Ala
    50                  55                  60
Cys Pro Ala Asp Cys Asp Pro Asn Thr Gln Ala Ser Cys Glu Cys Pro
65                  70                  75                  80
Glu Gly Tyr Ile Leu Asp Asp Gly Phe Ile Cys Thr Asp Ile Asp Glu
                85                  90                  95
Cys Glu Asn Gly Gly Phe Cys Ser Gly Val Cys His Asn Leu Pro Gly
            100                 105                 110
Thr Phe Glu Cys Ile Cys Gly Pro Asp Ser Ala Leu Ala Arg His Ile
        115                 120                 125
Gly Thr Asp Cys
    130
<210> 4
<211> 396
<212> DNA
<213> human
<400> 4
atgcttgggg tcctggtcct tggcgcgctg gccctggccg gcctggggtt ccccgacccg 60
tgcttcagag ccaactgcga gtaccagtgc cagcccctga accaaactag ctacctctgc 120
gtctgcgccg agggcttcgc gcccattccc cacgagccgc acaggtgcca gatgttttgc 180
aaccagactg cctgtccagc cgactgcgac cccaacaccc aggctagctg tgagtgccct 240
gaaggctaca tcctggacga cggtttcatc tgcacggaca tcgacgagtg cgaaaacggc 300
ggcttctgct ccggggtgtg ccacaacctc cccggtacct tcgagtgcat ctgcgggccc 360
gactcggccc ttgcccgcca cattggcacc gactgt 396

<210> 5
<211> 480
<212> PRT
<213> human
<400> 5
Met Leu Gly Val Leu Val Leu Gly Ala Leu Ala Leu Ala Gly Leu Gly
1               5                   10                  15
Phe Pro Ala Pro Ala Glu Pro Gln Pro Gly Gly Ser Gln Cys Val Glu
            20                  25                  30
His Asp Cys Phe Ala Leu Tyr Pro Gly Pro Ala Thr Phe Leu Asn Ala
        35                  40                  45
Ser Gln Ile Cys Asp Gly Leu Arg Gly His Leu Met Thr Val Arg Ser
    50                  55                  60
Ser Val Ala Ala Asp Val Ile Ser Leu Leu Leu Asn Gly Asp Gly Gly
65                  70                  75                  80
Val Gly Arg Arg Arg Leu Trp Ile Gly Leu Gln Leu Pro Pro Gly Cys
                85                  90                  95
Gly Asp Pro Lys Arg Leu Gly Pro Leu Arg Gly Phe Gln Trp Val Thr
            100                 105                 110

Gly Asp Asn Asn Thr Ser Tyr Ser Arg Trp Ala Arg Leu Asp Leu Asn
        115                 120                 125
Gly Ala Pro Leu Cys Gly Pro Leu Cys Val Ala Val Ser Ala Ala Glu
    130                 135                 140
Ala Thr Val Pro Ser Glu Pro Ile Trp Glu Glu Gln Gln Cys Glu Val
145                 150                 155                 160
Lys Ala Asp Gly Phe Leu Cys Glu Phe His Phe Pro Ala Thr Cys Arg
                165                 170                 175
Pro Leu Ala Val Glu Pro Gly Ala Ala Ala Ala Ala Val Ser Ile Thr
            180                 185                 190
Tyr Gly Thr Pro Phe Ala Ala Arg Gly Ala Asp Phe Gln Ala Leu Pro
        195                 200                 205
Val Gly Ser Ser Ala Ala Val Ala Pro Leu Gly Leu Gln Leu Met Cys
    210                 215                 220
Thr Ala Pro Pro Gly Ala Val Gln Gly His Trp Ala Arg Glu Ala Pro
225                 230                 235                 240
Gly Ala Trp Asp Cys Ser Val Glu Asn Gly Gly Cys Glu His Ala Cys
                245                 250                 255
Asn Ala Ile Pro Gly Ala Pro Arg Cys Gln Cys Pro Ala Gly Ala Ala
            260                 265                 270
Leu Gln Ala Asp Gly Arg Ser Cys Thr Ala Ser Ala Thr Gln Ser Cys
        275                 280                 285
Asn Asp Leu Cys Glu His Phe Cys Val Pro Asn Pro Asp Gln Pro Gly
    290                 295                 300
Ser Tyr Ser Cys Met Cys Glu Thr Gly Tyr Arg Leu Ala Ala Asp Gln
305                 310                 315                 320
His Arg Cys Glu Asp Val Asp Asp Cys Ile Leu Glu Pro Ser Pro Cys
                325                 330                 335
Pro Gln Arg Cys Val Asn Thr Gln Gly Gly Phe Glu Cys His Cys Tyr
            340                 345                 350
Pro Asn Tyr Asp Leu Val Asp Gly Glu Cys Val Glu Pro Val Asp Pro
        355                 360                 365
Cys Phe Arg Ala Asn Cys Glu Tyr Gln Cys Gln Pro Leu Asn Gln Thr
    370                 375                 380
Ser Tyr Leu Cys Val Cys Ala Glu Gly Phe Ala Pro Ile Pro His Glu
385                 390                 395                 400
Pro His Arg Cys Gln Met Phe Cys Asn Gln Thr Ala Cys Pro Ala Asp
                405                 410                 415
Cys Asp Pro Asn Thr Gln Ala Ser Cys Glu Cys Pro Glu Gly Tyr Ile
            420                 425                 430
Leu Asp Asp Gly Phe Ile Cys Thr Asp Ile Asp Glu Cys Glu Asn Gly
        435                 440                 445
Gly Phe Cys Ser Gly Val Cys His Asn Leu Pro Gly Thr Phe Glu Cys
    450                 455                 460
Ile Cys Gly Pro Asp Ser Ala Leu Val Arg His Ile Gly Thr Asp Cys
465                 470                 475                 480
<210> 6
<211> 1440

<212> DNA
<213> human
<400> 6
atgcttgggg tcctggtcct tggcgcgctg gccctggccg gcctggggtt ccccgcaccc 60
gcagagccgc agccgggtgg cagccagtgc gtcgagcacg actgcttcgc gctctacccg 120
ggccccgcga ccttcctcaa tgccagtcag atctgcgacg gactgcgggg ccacctaatg 180
acagtgcgct cctcggtggc tgccgatgtc atttccttgc tactgaacgg cgacggcggc 240
gttggccgcc ggcgcctctg gatcggcctg cagctgccac ccggctgcgg cgaccccaag 300
cgcctcgggc ccctgcgcgg cttccagtgg gttacgggag acaacaacac cagctatagc 360
aggtgggcac ggctcgacct caatggggct cccctctgcg gcccgttgtg cgtcgctgtc 420
tccgctgctg aggccactgt gcccagcgag ccgatctggg aggagcagca gtgcgaagtg 480
aaggccgatg gcttcctctg cgagttccac ttcccagcca cctgcaggcc actggctgtg 540
gagcccggcg ccgcggctgc cgccgtctcg atcacctacg gcaccccgtt cgcggcccgc 600
ggagcggact tccaggcgct gccggtgggc agctccgccg cggtggctcc cctcggctta 660
cagctaatgt gcaccgcgcc gcccggagcg gtccaggggc actgggccag ggaggcgccg 720
ggcgcttggg actgcagcgt ggagaacggc ggctgcgagc acgcgtgcaa tgcgatccct 780
ggggctcccc gctgccagtg cccagccggc gccgccctgc aggcagacgg gcgctcctgc 840
accgcatccg cgacgcagtc ctgcaacgac ctctgcgagc acttctgcgt tcccaacccc 900
gaccagccgg gctcctactc gtgcatgtgc gagaccggct accggctggc ggccgaccaa 960
caccggtgcg aggacgtgga tgactgcata ctggagccca gtccgtgtcc gcagcgctgt 1020
gtcaacacac agggtggctt cgagtgccac tgctacccta actacgacct ggtggacggc 1080
gagtgtgtgg agcccgtgga cccgtgcttc agagccaact gcgagtacca gtgccagccc 1140
ctgaaccaaa ctagctacct ctgcgtctgc gccgagggct tcgcgcccat tccccacgag 1200
ccgcacaggt gccagatgtt ttgcaaccag actgcctgtc cagccgactg cgaccccaac 1260
acccaggcta gctgtgagtg ccctgaaggc tacatcctgg acgacggttt catctgcacg 1320
gacatcgacg agtgcgaaaa cggcggcttc tgctccgggg tgtgccacaa cctccccggt 1380
accttcgagt gcatctgcgg gcccgactcg gcccttgtcc gccacattgg caccgactgt 1440
<210> 7
<211> 480
<212> PRT
<213> human
<400> 7
Met Leu Gly Val Leu Val Leu Gly Ala Leu Ala Leu Ala Gly Leu Gly
1               5                   10                  15
Phe Pro Ala Pro Ala Glu Pro Gln Pro Gly Gly Ser Gln Cys Val Glu
            20                  25                  30
His Asp Cys Phe Ala Leu Tyr Pro Gly Pro Ala Thr Phe Leu Asn Ala
        35                  40                  45
Ser Gln Ile Cys Asp Gly Leu Arg Gly His Leu Met Thr Val Arg Ser
    50                  55                  60
Ser Val Ala Ala Asp Val Ile Ser Leu Leu Leu Asn Gly Asp Gly Gly
65                  70                  75                  80
Val Gly Arg Arg Arg Leu Trp Ile Gly Leu Gln Leu Pro Pro Gly Cys
                85                  90                  95
Gly Asp Pro Lys Arg Leu Gly Pro Leu Arg Gly Phe Gln Trp Val Thr
            100                 105                 110
Gly Asp Asn Asn Thr Ser Tyr Ser Arg Trp Ala Arg Leu Asp Leu Asn
        115                 120                 125

Gly Ala Pro Leu Cys Gly Pro Leu Cys Val Ala Val Ser Ala Ala Glu
    130                 135                 140
Ala Thr Val Pro Ser Glu Pro Ile Trp Glu Glu Gln Gln Cys Glu Val
145                 150                 155                 160
Lys Ala Asp Gly Phe Leu Cys Glu Phe His Phe Pro Ala Thr Cys Arg
                165                 170                 175
Pro Leu Ala Val Glu Pro Gly Ala Ala Ala Ala Ala Val Ser Ile Thr
            180                 185                 190
Tyr Gly Thr Pro Phe Ala Ala Arg Gly Ala Asp Phe Gln Ala Leu Pro
        195                 200                 205
Val Gly Ser Ser Ala Ala Val Ala Pro Leu Gly Leu Gln Leu Met Cys
    210                 215                 220
Thr Ala Pro Pro Gly Ala Val Gln Gly His Trp Ala Arg Glu Ala Pro
225                 230                 235                 240
Gly Ala Trp Asp Cys Ser Val Glu Asn Gly Gly Cys Glu His Ala Cys
                245                 250                 255
Asn Ala Ile Pro Gly Ala Pro Arg Cys Gln Cys Pro Ala Gly Ala Ala
            260                 265                 270
Leu Gln Ala Asp Gly Arg Ser Cys Thr Ala Ser Ala Thr Gln Ser Cys
        275                 280                 285
Asn Asp Leu Cys Glu His Phe Cys Val Pro Asn Pro Asp Gln Pro Gly
    290                 295                 300
Ser Tyr Ser Cys Met Cys Glu Thr Gly Tyr Arg Leu Ala Ala Asp Gln
305                 310                 315                 320
His Arg Cys Glu Asp Val Asp Asp Cys Ile Leu Glu Pro Ser Pro Cys
                325                 330                 335
Pro Gln Arg Cys Val Asn Thr Gln Gly Gly Phe Glu Cys His Cys Tyr
            340                 345                 350
Pro Asn Tyr Asp Leu Val Asp Gly Glu Cys Val Glu Pro Val Asp Pro
        355                 360                 365
Cys Phe Arg Ala Asn Cys Glu Tyr Gln Cys Gln Pro Leu Asn Gln Thr
    370                 375                 380
Ser Tyr Leu Cys Val Cys Ala Glu Gly Phe Ala Pro Ile Pro His Glu
385                 390                 395                 400
Pro His Arg Cys Gln Met Phe Cys Asn Gln Thr Ala Cys Pro Ala Asp
                405                 410                 415
Cys Asp Pro Asn Thr Gln Ala Ser Cys Glu Cys Pro Glu Gly Tyr Ile
            420                 425                 430
Leu Asp Asp Gly Phe Ile Cys Thr Asp Ile Asp Glu Cys Glu Asn Gly
        435                 440                 445
Gly Phe Cys Ser Gly Val Cys His Asn Leu Pro Gly Thr Phe Glu Cys
    450                 455                 460
Ile Cys Gly Pro Asp Ser Ala Leu Ala Arg His Ile Gly Thr Asp Cys
465                 470                 475                 480
<210> 8
<211> 1440
<212> DNA
<213> human

<400> 8
atgcttgggg tcctggtcct tggcgcgctg gccctggccg gcctggggtt ccccgcaccc 60
gcagagccgc agccgggtgg cagccagtgc gtcgagcacg actgcttcgc gctctacccg 120
ggccccgcga ccttcctcaa tgccagtcag atctgcgacg gactgcgggg ccacctaatg 180
acagtgcgct cctcggtggc tgccgatgtc atttccttgc tactgaacgg cgacggcggc 240
gttggccgcc ggcgcctctg gatcggcctg cagctgccac ccggctgcgg cgaccccaag 300
cgcctcgggc ccctgcgcgg cttccagtgg gttacgggag acaacaacac cagctatagc 360
aggtgggcac ggctcgacct caatggggct cccctctgcg gcccgttgtg cgtcgctgtc 420
tccgctgctg aggccactgt gcccagcgag ccgatctggg aggagcagca gtgcgaagtg 480
aaggccgatg gcttcctctg cgagttccac ttcccagcca cctgcaggcc actggctgtg 540
gagcccggcg ccgcggctgc cgccgtctcg atcacctacg gcaccccgtt cgcggcccgc 600
ggagcggact tccaggcgct gccggtgggc agctccgccg cggtggctcc cctcggctta 660
cagctaatgt gcaccgcgcc gcccggagcg gtccaggggc actgggccag ggaggcgccg 720
ggcgcttggg actgcagcgt ggagaacggc ggctgcgagc acgcgtgcaa tgcgatccct 780
ggggctcccc gctgccagtg cccagccggc gccgccctgc aggcagacgg gcgctcctgc 840
accgcatccg cgacgcagtc ctgcaacgac ctctgcgagc acttctgcgt tcccaacccc 900
gaccagccgg gctcctactc gtgcatgtgc gagaccggct accggctggc ggccgaccaa 960
caccggtgcg aggacgtgga tgactgcata ctggagccca gtccgtgtcc gcagcgctgt 1020
gtcaacacac agggtggctt cgagtgccac tgctacccta actacgacct ggtggacggc 1080
gagtgtgtgg agcccgtgga cccgtgcttc agagccaact gcgagtacca gtgccagccc 1140
ctgaaccaaa ctagctacct ctgcgtctgc gccgagggct tcgcgcccat tccccacgag 1200
ccgcacaggt gccagatgtt ttgcaaccag actgcctgtc cagccgactg cgaccccaac 1260
acccaggcta gctgtgagtg ccctgaaggc tacatcctgg acgacggttt catctgcacg 1320
gacatcgacg agtgcgaaaa cggcggcttc tgctccgggg tgtgccacaa cctccccggt 1380
accttcgagt gcatctgcgg gcccgactcg gcccttgccc gccacattgg caccgactgt 1440
<210> 9
<211> 516
<212> PRT
<213> human
<400> 9
Met Leu Gly Val Leu Val Leu Gly Ala Leu Ala Leu Ala Gly Leu Gly
1               5                   10                  15
Phe Pro Ala Pro Ala Glu Pro Gln Pro Gly Gly Ser Gln Cys Val Glu
            20                  25                  30
His Asp Cys Phe Ala Leu Tyr Pro Gly Pro Ala Thr Phe Leu Asn Ala
        35                  40                  45
Ser Gln Ile Cys Asp Gly Leu Arg Gly His Leu Met Thr Val Arg Ser
    50                  55                  60
Ser Val Ala Ala Asp Val Ile Ser Leu Leu Leu Asn Gly Asp Gly Gly
65                  70                  75                  80
Val Gly Arg Arg Arg Leu Trp Ile Gly Leu Gln Leu Pro Pro Gly Cys
                85                  90                  95
Gly Asp Pro Lys Arg Leu Gly Pro Leu Arg Gly Phe Gln Trp Val Thr
            100                 105                 110
Gly Asp Asn Asn Thr Ser Tyr Ser Arg Trp Ala Arg Leu Asp Leu Asn
        115                 120                 125
Gly Ala Pro Leu Cys Gly Pro Leu Cys Val Ala Val Ser Ala Ala Glu
    130                 135                 140

Ala Thr Val Pro Ser Glu Pro Ile Trp Glu Glu Gln Gln Cys Glu Val
145                 150                 155                 160
Lys Ala Asp Gly Phe Leu Cys Glu Phe His Phe Pro Ala Thr Cys Arg
                165                 170                 175
Pro Leu Ala Val Glu Pro Gly Ala Ala Ala Ala Ala Val Ser Ile Thr
            180                 185                 190
Tyr Gly Thr Pro Phe Ala Ala Arg Gly Ala Asp Phe Gln Ala Leu Pro
        195                 200                 205
Val Gly Ser Ser Ala Ala Val Ala Pro Leu Gly Leu Gln Leu Met Cys
    210                 215                 220
Thr Ala Pro Pro Gly Ala Val Gln Gly His Trp Ala Arg Glu Ala Pro
225                 230                 235                 240
Gly Ala Trp Asp Cys Ser Val Glu Asn Gly Gly Cys Glu His Ala Cys
                245                 250                 255
Asn Ala Ile Pro Gly Ala Pro Arg Cys Gln Cys Pro Ala Gly Ala Ala
            260                 265                 270
Leu Gln Ala Asp Gly Arg Ser Cys Thr Ala Ser Ala Thr Gln Ser Cys
        275                 280                 285
Asn Asp Leu Cys Glu His Phe Cys Val Pro Asn Pro Asp Gln Pro Gly
    290                 295                 300
Ser Tyr Ser Cys Met Cys Glu Thr Gly Tyr Arg Leu Ala Ala Asp Gln
305                 310                 315                 320
His Arg Cys Glu Asp Val Asp Asp Cys Ile Leu Glu Pro Ser Pro Cys
                325                 330                 335
Pro Gln Arg Cys Val Asn Thr Gln Gly Gly Phe Glu Cys His Cys Tyr
            340                 345                 350
Pro Asn Tyr Asp Leu Val Asp Gly Glu Cys Val Glu Pro Val Asp Pro
        355                 360                 365
Cys Phe Arg Ala Asn Cys Glu Tyr Gln Cys Gln Pro Leu Asn Gln Thr
    370                 375                 380
Ser Tyr Leu Cys Val Cys Ala Glu Gly Phe Ala Pro Ile Pro His Glu
385                 390                 395                 400
Pro His Arg Cys Gln Met Phe Cys Asn Gln Thr Ala Cys Pro Ala Asp
                405                 410                 415
Cys Asp Pro Asn Thr Gln Ala Ser Cys Glu Cys Pro Glu Gly Tyr Ile
            420                 425                 430
Leu Asp Asp Gly Phe Ile Cys Thr Asp Ile Asp Glu Cys Glu Asn Gly
        435                 440                 445
Gly Phe Cys Ser Gly Val Cys His Asn Leu Pro Gly Thr Phe Glu Cys
    450                 455                 460
Ile Cys Gly Pro Asp Ser Ala Leu Val Arg His Ile Gly Thr Asp Cys
465                 470                 475                 480
Asp Ser Gly Lys Val Asp Gly Gly Asp Ser Gly Ser Gly Glu Pro Pro
                485                 490                 495
Pro Ser Pro Thr Pro Gly Ser Thr Leu Thr Pro Pro Ala Val Gly Leu
            500                 505                 510
Val His Ser Gly
        515

<210> 10
<211> 1548
<212> DNA
<213> human
<400> 10
atgcttgggg tcctggtcct tggcgcgctg gccctggccg gcctggggtt ccccgcaccc 60
gcagagccgc agccgggtgg cagccagtgc gtcgagcacg actgcttcgc gctctacccg 120
ggccccgcga ccttcctcaa tgccagtcag atctgcgacg gactgcgggg ccacctaatg 180
acagtgcgct cctcggtggc tgccgatgtc atttccttgc tactgaacgg cgacggcggc 240
gttggccgcc ggcgcctctg gatcggcctg cagctgccac ccggctgcgg cgaccccaag 300
cgcctcgggc ccctgcgcgg cttccagtgg gttacgggag acaacaacac cagctatagc 360
aggtgggcac ggctcgacct caatggggct cccctctgcg gcccgttgtg cgtcgctgtc 420
tccgctgctg aggccactgt gcccagcgag ccgatctggg aggagcagca gtgcgaagtg 480
aaggccgatg gcttcctctg cgagttccac ttcccagcca cctgcaggcc actggctgtg 540
gagcccggcg ccgcggctgc cgccgtctcg atcacctacg gcaccccgtt cgcggcccgc 600
ggagcggact tccaggcgct gccggtgggc agctccgccg cggtggctcc cctcggctta 660
cagctaatgt gcaccgcgcc gcccggagcg gtccaggggc actgggccag ggaggcgccg 720
ggcgcttggg actgcagcgt ggagaacggc ggctgcgagc acgcgtgcaa tgcgatccct 780
ggggctcccc gctgccagtg cccagccggc gccgccctgc aggcagacgg gcgctcctgc 840
accgcatccg cgacgcagtc ctgcaacgac ctctgcgagc acttctgcgt tcccaacccc 900
gaccagccgg gctcctactc gtgcatgtgc gagaccggct accggctggc ggccgaccaa 960
caccggtgcg aggacgtgga tgactgcata ctggagccca gtccgtgtcc gcagcgctgt 1020
gtcaacacac agggtggctt cgagtgccac tgctacccta actacgacct ggtggacggc 1080
gagtgtgtgg agcccgtgga cccgtgcttc agagccaact gcgagtacca gtgccagccc 1140
ctgaaccaaa ctagctacct ctgcgtctgc gccgagggct tcgcgcccat tccccacgag 1200
ccgcacaggt gccagatgtt ttgcaaccag actgcctgtc cagccgactg cgaccccaac 1260
acccaggcta gctgtgagtg ccctgaaggc tacatcctgg acgacggttt catctgcacg 1320
gacatcgacg agtgcgaaaa cggcggcttc tgctccgggg tgtgccacaa cctccccggt 1380
accttcgagt gcatctgcgg gcccgactcg gcccttgtcc gccacattgg caccgactgt 1440
gactccggca aggtggacgg tggcgacagc ggctctggcg agcccccgcc cagcccgacg 1500
cccggctcca ccttgactcc tccggccgtg gggctcgtgc attcgggc 1548
<210> 11
<211> 516
<212> PRT
<213> human
<400> 11
Met Leu Gly Val Leu Val Leu Gly Ala Leu Ala Leu Ala Gly Leu Gly
1               5                   10                  15
Phe Pro Ala Pro Ala Glu Pro Gln Pro Gly Gly Ser Gln Cys Val Glu
            20                  25                  30
His Asp Cys Phe Ala Leu Tyr Pro Gly Pro Ala Thr Phe Leu Asn Ala
        35                  40                  45
Ser Gln Ile Cys Asp Gly Leu Arg Gly His Leu Met Thr Val Arg Ser
    50                  55                  60
Ser Val Ala Ala Asp Val Ile Ser Leu Leu Leu Asn Gly Asp Gly Gly
65                  70                  75                  80
Val Gly Arg Arg Arg Leu Trp Ile Gly Leu Gln Leu Pro Pro Gly Cys
                85                  90                  95

Gly Asp Pro Lys Arg Leu Gly Pro Leu Arg Gly Phe Gln Trp Val Thr
            100                 105                 110
Gly Asp Asn Asn Thr Ser Tyr Ser Arg Trp Ala Arg Leu Asp Leu Asn
        115                 120                 125
Gly Ala Pro Leu Cys Gly Pro Leu Cys Val Ala Val Ser Ala Ala Glu
    130                 135                 140
Ala Thr Val Pro Ser Glu Pro Ile Trp Glu Glu Gln Gln Cys Glu Val
145                 150                 155                 160
Lys Ala Asp Gly Phe Leu Cys Glu Phe His Phe Pro Ala Thr Cys Arg
                165                 170                 175
Pro Leu Ala Val Glu Pro Gly Ala Ala Ala Ala Ala Val Ser Ile Thr
            180                 185                 190
Tyr Gly Thr Pro Phe Ala Ala Arg Gly Ala Asp Phe Gln Ala Leu Pro
        195                 200                 205
Val Gly Ser Ser Ala Ala Val Ala Pro Leu Gly Leu Gln Leu Met Cys
    210                 215                 220
Thr Ala Pro Pro Gly Ala Val Gln Gly His Trp Ala Arg Glu Ala Pro
225                 230                 235                 240
Gly Ala Trp Asp Cys Ser Val Glu Asn Gly Gly Cys Glu His Ala Cys
                245                 250                 255
Asn Ala Ile Pro Gly Ala Pro Arg Cys Gln Cys Pro Ala Gly Ala Ala
            260                 265                 270
Leu Gln Ala Asp Gly Arg Ser Cys Thr Ala Ser Ala Thr Gln Ser Cys
        275                 280                 285
Asn Asp Leu Cys Glu His Phe Cys Val Pro Asn Pro Asp Gln Pro Gly
    290                 295                 300
Ser Tyr Ser Cys Met Cys Glu Thr Gly Tyr Arg Leu Ala Ala Asp Gln
305                 310                 315                 320
His Arg Cys Glu Asp Val Asp Asp Cys Ile Leu Glu Pro Ser Pro Cys
                325                 330                 335
Pro Gln Arg Cys Val Asn Thr Gln Gly Gly Phe Glu Cys His Cys Tyr
            340                 345                 350
Pro Asn Tyr Asp Leu Val Asp Gly Glu Cys Val Glu Pro Val Asp Pro
        355                 360                 365
Cys Phe Arg Ala Asn Cys Glu Tyr Gln Cys Gln Pro Leu Asn Gln Thr
    370                 375                 380
Ser Tyr Leu Cys Val Cys Ala Glu Gly Phe Ala Pro Ile Pro His Glu
385                 390                 395                 400
Pro His Arg Cys Gln Met Phe Cys Asn Gln Thr Ala Cys Pro Ala Asp
                405                 410                 415
Cys Asp Pro Asn Thr Gln Ala Ser Cys Glu Cys Pro Glu Gly Tyr Ile
            420                 425                 430
Leu Asp Asp Gly Phe Ile Cys Thr Asp Ile Asp Glu Cys Glu Asn Gly
        435                 440                 445
Gly Phe Cys Ser Gly Val Cys His Asn Leu Pro Gly Thr Phe Glu Cys
    450                 455                 460
Ile Cys Gly Pro Asp Ser Ala Leu Ala Arg His Ile Gly Thr Asp Cys
465                 470                 475                 480

Asp Ser Gly Lys Val Asp Gly Gly Asp Ser Gly Ser Gly Glu Pro Pro
                485                 490                 495
Pro Ser Pro Thr Pro Gly Ser Thr Leu Thr Pro Pro Ala Val Gly Leu
            500                 505                 510
Val His Ser Gly
        515
<210> 12
<211> 1548
<212> DNA
<213> human
<400> 12
atgcttgggg tcctggtcct tggcgcgctg gccctggccg gcctggggtt ccccgcaccc 60
gcagagccgc agccgggtgg cagccagtgc gtcgagcacg actgcttcgc gctctacccg 120
ggccccgcga ccttcctcaa tgccagtcag atctgcgacg gactgcgggg ccacctaatg 180
acagtgcgct cctcggtggc tgccgatgtc atttccttgc tactgaacgg cgacggcggc 240
gttggccgcc ggcgcctctg gatcggcctg cagctgccac ccggctgcgg cgaccccaag 300
cgcctcgggc ccctgcgcgg cttccagtgg gttacgggag acaacaacac cagctatagc 360
aggtgggcac ggctcgacct caatggggct cccctctgcg gcccgttgtg cgtcgctgtc 420
tccgctgctg aggccactgt gcccagcgag ccgatctggg aggagcagca gtgcgaagtg 480
aaggccgatg gcttcctctg cgagttccac ttcccagcca cctgcaggcc actggctgtg 540
gagcccggcg ccgcggctgc cgccgtctcg atcacctacg gcaccccgtt cgcggcccgc 600
ggagcggact tccaggcgct gccggtgggc agctccgccg cggtggctcc cctcggctta 660
cagctaatgt gcaccgcgcc gcccggagcg gtccaggggc actgggccag ggaggcgccg 720
ggcgcttggg actgcagcgt ggagaacggc ggctgcgagc acgcgtgcaa tgcgatccct 780
ggggctcccc gctgccagtg cccagccggc gccgccctgc aggcagacgg gcgctcctgc 840
accgcatccg cgacgcagtc ctgcaacgac ctctgcgagc acttctgcgt tcccaacccc 900
gaccagccgg gctcctactc gtgcatgtgc gagaccggct accggctggc ggccgaccaa 960
caccggtgcg aggacgtgga tgactgcata ctggagccca gtccgtgtcc gcagcgctgt 1020
gtcaacacac agggtggctt cgagtgccac tgctacccta actacgacct ggtggacggc 1080
gagtgtgtgg agcccgtgga cccgtgcttc agagccaact gcgagtacca gtgccagccc 1140
ctgaaccaaa ctagctacct ctgcgtctgc gccgagggct tcgcgcccat tccccacgag 1200
ccgcacaggt gccagatgtt ttgcaaccag actgcctgtc cagccgactg cgaccccaac 1260
acccaggcta gctgtgagtg ccctgaaggc tacatcctgg acgacggttt catctgcacg 1320
gacatcgacg agtgcgaaaa cggcggcttc tgctccgggg tgtgccacaa cctccccggt 1380
accttcgagt gcatctgcgg gcccgactcg gcccttgccc gccacattgg caccgactgt 1440
gactccggca aggtggacgg tggcgacagc ggctctggcg agcccccgcc cagcccgacg 1500
cccggctcca ccttgactcc tccggccgtg gggctcgtgc attcgggc 1548
<210> 13
<211> 21
<212> DNA
<213> Artificial Sequence
<220>
<223> Description of Artificial Sequence: Synthetic DNA
<400> 13
aatgtggcgg gcaagggccg a 21

## Description

### TECHNICAL FIELD

The present invention relates to a therapeutically effective agent for therapy and/or improvement of disseminated intravascular coagulation, which comprises thrombomodulin as an active ingredient. The present invention also relates to a method for selecting patients suffering from disseminated intravascular coagulation, to whom thrombomodulin is to be administered.

### BACKGROUND ART

Disseminated intravascular coagulation (hereinafter abbreviated as DIC, at times) is one type of coagulation disease, and is a disease whereby large quantities of blood coagulation-accelerating substances are generated as a result of tissue damage caused by various diseases, so that the function of a coagulation system is excessively accelerated, and small thrombuses are formed in generalized blood vessel and they clog small vessels, and at the same time, thrombocytes or coagulation factors necessary for the control of bleeding are consumed, thereby causing clotting abnormality. In the case of DIC, blood coagulation factors are accumulated in the microvessels of kidney and lung so as to cause ischaemia, and as a result, circulatory disorder and shock take place in many cases. Thereby, high respiration rate, dyspnea, tachycardia, bradycardia, coldness of extremities, oliguria, convulsion, and the like may be observed. In some cases, such shock disease may result in sudden death. At the same time, consumption of coagulation factors and thrombocytes leads to bleeding, so that purpura on the skin, petechiae, hemoptysis, hematemesis, hematuria, bloody stool, nasal bleeding, and the like may take place. As a DIC therapeutic agent, heparin, antithrombin (which is also referred to as antithrombin III; hereinafter abbreviated as AT or AT III, at times.), and the like have been used.

On the other hand, thrombomodulin has been known as a substance that acts to specifically bind to thrombin so as to inhibit the blood coagulation activity thereof, and at the same time, acts to significantly promote the ability of the thrombin to activate protein C. Thrombomodulin has also been known to have strong blood coagulation-inhibiting action. It has also been known that thrombomodulin extends the coagulation time by thrombin, or that it suppresses platelet aggregation due to thrombin. Protein C is a vitamin K-dependent protein that plays an important role in a blood coagulation fibrinolytic system. Protein C is activated by the action of thrombin, so that it becomes activated protein C. It has been known that the activated protein C deactivates an activated blood coagulation factor V and an activated blood coagulation factor VIII, and that it is involved in generation of a plasminogen activator having thrombolytic action (Non-Patent Document 1). Accordingly, it has been considered that thrombomodulin promotes the activation of protein C by thrombin, and thus that this is useful as an anticoagulant or a thrombolytic agent. Also, it has been reported in an animal experiment that thrombomodulin is effective for therapy or prevention of diseases associated with acceleration of coagulation (Non-Patent Document 2).

Conventionally, thrombomodulin has been discovered and obtained as a glycoprotein that is expressed on the vascular endothelial cells of various animal species, including humans as typical examples, and thereafter has been successfully cloned. That is to say, a human thrombomodulin precursor gene containing a signal peptide has been cloned from a human lung cDNA library by genetic engineering, and all the gene sequences of thrombomodulin have been analyzed. As a result, an amino acid sequence consisting of 575 residues containing a signal peptide (in general, 18 amino acid residues are exemplified) has been clarified (Patent Document 1). It has been known that a mature thrombomodulin, from which the signal peptide has been cleaved, is composed of 5 regions, namely, an N-terminal region (amino acids 1-226: this is the position determined when the signal peptide is assumed to consist of 18 amino acid residues, and the same holds true for other regions), a region having six EGF-like structures (amino acids 227-462), an O-linked glycosylation region (amino acids 463-498), a transmembrane region (amino acids 499-521), and an intracytoplasmic region (amino acids 522-557), from the N-terminal side of the mature peptide. It has also been known that, among the six EGF-like structures, the 4^{th}, 5^{th}, and 6^{th} EGF-like structure portions from the N-terminal side (that is, minimal units of activity) mainly have the same activity as that of the entire-length thrombomodulin (Non-Patent Document 3).

Unless a surfactant is present, the entire-length thrombomodulin is hardly dissolved. Thus, addition of a surfactant is necessary for producing a thrombomodulin preparation. In contrast, there is also a soluble thrombomodulin that can be fully dissolved even in the absence of a surfactant. The soluble thrombomodulin may be prepared by removing at least a part of the transmembrane region or the entire transmembrane region. For example, it has been confirmed that a soluble thrombomodulin consisting of only 3 regions, namely, an N-terminal region, a region having six EGF-like structures, and an O-linked glycosylation region (that is, a soluble thrombomodulin having an amino acid sequence consisting of amino acids 19-516 of SEQ ID NO: 9), can be obtained by applying recombination techniques, and that this recombinant soluble thrombomodulin has the same activity as that of a native thrombomodulin (Patent Document 1). In addition, there are some other reports regarding soluble thrombomodulins (Patent Documents 2 to 9). Also, a human urine-derived soluble thrombomodulin and the like have been exemplified as native thrombomodulins (Patent Documents 10 and 11).

As recognized in many cases, as a result of spontaneous mutations or mutations occurring when thrombomodulins are obtained, polymorphic mutations have been found even in human genes. At present, thrombomodulin genes in which the amino acid at position 473 of a human thrombomodulin precursor having the aforementioned amino acid sequence consisting of 575 amino acid residues is converted to Val or Ala have been identified. In a nucleotide sequence encoding this amino acid, the nucleotide at position 1418 is converted to T or C (Non-Patent Document 4). However, the two thrombomodulins are completely identical in terms of their activity and physical properties. Thus, it can be considered that they are substantially identical.

It has been reported that thrombomodulin has effects on the therapy of DIC (Non-Patent Document 5). In addition to the aforementioned intended uses, it is anticipated that thrombomodulin will be used in the therapy and prevention of various diseases such as acute coronary syndrome (ACS), thrombosis, peripheral vascular obstruction, arteriosclerosis obliterans, vasculitis, functional disorder occurring after heart surgery, complication caused by organ transplantation, angina pectoris, transient ischemic attack, toxemia of pregnancy, diabetes, liver VOD (liver veno-occlusive disease; e.g. fulminant hepatitis, veno occlusive disease of liver occurring after bone marrow transplantation), deep venous thrombosis (DVT), septicemia, and adult respiratory distress syndrome (ARDS).
Patent Document 1: JP Patent Publication (Kokai) No. 64-6219 A (1989)
Patent Document 2: JP Patent Publication (Kokai) No. 2-255699 A (1990)
Patent Document 3: JP Patent Publication (Kokai) No. 3-133380 A (1991)
Patent Document 4: JP Patent Publication (Kokai) No. 3-259084 A (1991)
Patent Document 5: JP Patent Publication (Kokai) No. 4-210700 A (1992)
Patent Document 6: JP Patent Publication (Kokai) No. 5-213998 A (1993)
Patent Document 7: WO92/00325
Patent Document 8: WO92/03149
Patent Document 9: WO93/15755
Patent Document 10: JP Patent Publication (Kokai) No. 3-86900 A (1991)
Patent Document 11: JP Patent Publication (Kokai) No. 3-218399 A (1991)
Non-Patent Document 1: Koji Suzuki, Igaku no Ayumi (Progression of Medicines), Vol. 125, p. 901 (1983)
Non-Patent Document 2: K. Gomi et al., Blood 75.,1396-1399 (1990)
Non-Patent Document 3: M. Zushi et al., J. Biol. Chem., 246, 10351-10353 (1989)
Non-Patent Document 4: D. Z. Wen et al., Biochemistry, 26, 4350-4357 (1987)
Non-Patent Document 5: S. M. Bates et al., Br. J. of Pharmacol., 144, 1017-1028 (2005)

### DISCLOSURE OF THE INVENTION

### Problems to be Solved by the Invention

It is an object of the present invention to provide a therapeutically effective agent for therapy and/or improvement of DIC, or a method for treating and/or improving DIC. It is another object of the present invention to provide a method for selecting patients suffering from disseminated intravascular coagulation, to whom thrombomodulin is to be administered.

### Means for Solving the Problems

As means for treating and/or improving DIC, an anticoagulant therapy using an anticoagulant or a substitution therapy using concentrated thrombocytes or fresh frozen plasma has generally been applied. The essential symptom of DIC is excessive activation of a blood coagulation system. Thus, the anticoagulant therapy for suppressing such excessive activation has been widely carried out in parallel with the treatment of underlying disease. In the therapy and/or improvement of DIC, heparin and/or AT preparations have been generally used. It has been considered that the anticoagulant action of heparin is expressed by significantly promoting the coagulation factor-inhibiting rate of AT (Majerus PW, Tollefsen DM. "Coagulants, thrombolytic agents, and anti-platelet agents" In: edited by Shuji Takaori, Hideomi Fukuda, and Akinori Akaike, Goodman & Gilman's The Pharmacological Basis of Therapeutics (10th ed), Tokyo, Hirokawa Shoten, Co., 2003, pp. 1937-1963). Thus, heparin has been used for patients having a relatively high plasma AT activity such as a plasma AT activity of approximately 80% (Kenji Okajima, "Hanshusei kekkannai gyoko shokogun to tazoki fuzen (Disseminated intravascular coagulation syndrome and multiple organ failure)," Iyaku (Medicine and Drug) Journal Co., Ltd.). Moreover, an AT preparation is a typical agent administered to patients having a plasma AT activity of, for example, approximately 70% or less (package leaflet for Anthrobin P/Neuart, "Usage and Dose"). A method for treating and/or improving DIC by enhancing plasma AT activity to promote anticoagulant action has been mainly adopted. Furthermore, heparin may be used together with such an AT preparation at that time, as necessary.

Hence, in order to treat and/or improve DIC, it is preferred that supplement is performed in such as way that the plasma AT activity of DIG patients is kept at a level which is at least 70% or more. Further, an increase in plasma AT activity is generally considered to be important to treat and/or improve DIC. Under such circumstances, the present inventors have found that, unexpectedly, high effectiveness can be achieved in patients having a low plasma AT activity of less than 50%, thereby completing the present invention. That is, even underestimating, the preparation is highly effective for DIC patients having a plasma AT activity of less than 50%. Thus, the inventors have obtained extraordinary results that could not have been conceived of even by persons skilled in the art.

AT preparations are produced from valuable human blood used as a raw material. If such AT preparations are actually administered to patients, it is inevitable to perfectly eliminate a risk of dissemination of infectious disease caused by the use of human blood as a raw material (a package leaflet for Anthrobin P/Neuart, "Instructions for Use"). Thus, AT preparations have been problematic to some extent Furthermore, AT used as a blood preparation has also been problematic in terms of expensiveness. However, the present invention is able to provide a preferred agent, or an agent for therapy and/or improvement of DIC, which solves the aforementioned problems.

Specifically, the present invention includes the following features:
[1] An agent for therapy and/or improvement of disseminated intravascular coagulation comprising thrombomodulin as an active ingredient, which is administered to patients suffering from disseminated intravascular coagulation who have a plasma antithrombin activity of less than 50%;
[2] The agent for therapy and/or improvement of disseminated intravascular coagulation according to [1] above, wherein said thrombomodulin is soluble thrombomodulin;
[3] The agent for therapy and/or improvement of disseminated intravascular coagulation according to [1] or [2] above, wherein said thrombomodulin is a peptide obtained from transformant cells prepared by transfecting host cells with DNA encoding the amino acid sequence as shown in SEQ ID NO: 1, 3, 5, 7, 9, or 11;
   [3-2] The agent for therapy and/or improvement of disseminated intravascular coagulation according to [1] or [2] above, wherein said thrombomodulin is a peptide obtained from transformant cells prepared by transfecting host cells with DNA encoding the amino acid sequence as shown in SEQ ID NO: 1 or 3;
   [3-3] The agent for therapy and/or improvement of disseminated intravascular coagulation according to [1] or [2] above, wherein said thrombomodulin is a peptide obtained from transformant cells prepared by transfecting host cells with DNA encoding the amino acid sequence as shown in SEQ ID NO: 5 or 7;
   [3-4] The agent for therapy and/or improvement of disseminated intravascular coagulation according to [1] or [2] above, wherein said thrombomodulin is a peptide obtained from transformant cells prepared by transfecting host cells with DNA encoding the amino acid sequence as shown in SEQ ID NO: 9 or 11;
[4] The agent for therapy and/or improvement of disseminated intravascular coagulation according to any one of [1], [2], and [3-2] above, wherein said thrombomodulin is a peptide having a sequence consisting of amino acids at positions 19 to 132 of the amino acid sequence as shown in SEQ ID NO: 1 or 3, a peptide having a homologous mutation sequence of said sequence and having thrombomodulin activity, or a mixture thereof;
   [4-2] The agent for therapy and/or improvement of disseminated intravascular coagulation according to any one of [1], [2], and [3-2] above, wherein said thrombomodulin is a peptide having a sequence consisting of amino acids at positions 19 to 132 of the amino acid sequence as shown in SEQ ID NO: 1 or 3, or a peptide having a homologous mutation sequence of said sequence and having thrombomodulin activity;
   [4-3] The agent for therapy and/or improvement of disseminated intravascular coagulation according to any one of [1], [2], and [3-2] above, wherein said thrombomodulin is a peptide having a sequence consisting of amino acids at positions 19 to 132 of the amino acid sequence as shown in SEQ ID NO: 1 or 3;
   [4-4] The agent for therapy and/or improvement of disseminated intravascular coagulation according to any one of [1], [2], and [3-2] above, wherein said thrombomodulin is a peptide having a sequence consisting of amino acids at positions 19 to 132 or at positions 17 to 132 of the amino acid sequence as shown in SEQ ID NO: 1 or 3, a peptide having a homologous mutation sequence of said sequence and having thrombomodulin activity, or a mixture thereof;
   [4-5] The agent for therapy and/or improvement of disseminated intravascular coagulation according to any one of [1], [2], and [3-2] above, wherein said thrombomodulin is a peptide having a sequence consisting of amino acids at positions 19 to 132 or at positions 17 to 132 of the amino acid sequence as shown in SEQ ID NO: 1 or 3, or a mixture thereof;
   [4-6] The agent for therapy and/or improvement of disseminated intravascular coagulation according to any one of [1], [2], and [3-2] above, wherein said thrombomodulin is a peptide having a sequence consisting of amino acids at positions 19 to 132 or at positions 17 to 132 of the amino acid sequence as shown in SEQ ID NO: 1, or a mixture thereof;
   [4-7] The agent for therapy and/or improvement of disseminated intravascular coagulation according to any one of [1], [2], and [3-2] above, wherein said thrombomodulin is a peptide having a sequence consisting of amino acids at positions 19 to 132 or at positions 17 to 132 of the amino acid sequence as shown in SEQ ID NO: 3, or a mixture thereof;
   [4-8] The agent for therapy and/or improvement of disseminated intravascular coagulation according to any one of [1], [2], and [3-2] above, wherein said thrombomodulin is a peptide consisting of a sequence consisting of amino acids at positions 19 to 132 or at positions 17 to 132 of the amino acid sequence as shown in SEQ ID NO: 1, or a mixture thereof;
   [4-9] The agent for therapy and/or improvement of disseminated intravascular coagulation according to any one of [1], [2], and [3-2] above, wherein said thrombomodulin is a peptide consisting of a sequence consisting of amino acids at positions 19 to 132 or at positions 17 to 132 of the amino acid sequence as shown in SEQ ID NO: 3, or a mixture thereof;
[5] The agent for therapy and/or improvement of disseminated intravascular coagulation according to any one of [1], [2], and [3-3] above, wherein said thrombomodulin is a peptide having a sequence consisting of amino acids at positions 19 to 480 of the amino acid sequence as shown in SEQ ID NO: 5 or 7, a peptide having a homologous mutation sequence of said sequence and having thrombomodulin activity, or a mixture thereof;
   [5-2] The agent for therapy and/or improvement of disseminated intravascular coagulation according to any one of [1], [2], and [3-3] above, wherein said thrombomodulin is a peptide having a sequence consisting of amino acids at positions 19 to 480 of the amino acid sequence as shown in SEQ ID NO: 5 or 7, or a peptide having a homologous mutation sequence of said sequence and having thrombomodulin activity;
   [5-3] The agent for therapy and/or improvement of disseminated intravascular coagulation according to any one of [1], [2], and [3-3] above, wherein said thrombomodulin is a peptide having a sequence consisting of amino acids at positions 19 to 480 of the amino acid sequence as shown in SEQ ID NO: 5 or 7;
   [5-4] The agent for therapy and/or improvement of disseminated intravascular coagulation according to any one of [1], [2], and [3-3] above, wherein said thrombomodulin is a peptide having a sequence consisting of amino acids at positions 19 to 480 or at positions 17 to 480 of the amino acid sequence as shown in SEQ ID NO: 5 or 7, a peptide having a homologous mutation sequence of said sequence and having thrombomodulin activity, or a mixture thereof;
   [5-5] The agent for therapy and/or improvement of disseminated intravascular coagulation according to any one of [1], [2], and [3-3] above, wherein said thrombomodulin is a peptide having a sequence consisting of amino acids at positions 19 to 480 or at positions 17 to 480 of the amino acid sequence as shown in SEQ ID NO: 5 or 7, or a mixture thereof;
   [5-6] The agent for therapy and/or improvement of disseminated intravascular coagulation according to any one of [1], [2], and [3-3] above, wherein said thrombomodulin is a peptide having a sequence consisting of amino acids at positions 19 to 480 or at positions 17 to 480 of the amino acid sequence as shown in SEQ ID NO: 5, or a mixture thereof;
   [5-7] The agent for therapy and/or improvement of disseminated intravascular coagulation according to any one of [1], [2], and [3-3] above, wherein said thrombomodulin is a peptide having a sequence consisting of amino acids at positions 19 to 480 or at positions 17 to 480 of the amino acid sequence as shown in SEQ ID NO: 7, or a mixture thereof;
   [5-8] The agent for therapy and/or improvement of disseminated intravascular coagulation according to any one of [1], [2], and [3-3] above, wherein said thrombomodulin is a peptide consisting of a sequence consisting of amino acids at positions 19 to 480 or at positions 17 to 480 of the amino acid sequence as shown in SEQ ID NO: 5, or a mixture thereof;
   [5-9] The agent for therapy and/or improvement of disseminated intravascular coagulation according to any one of [1], [2], and [3-3] above, wherein said thrombomodulin is a peptide consisting of a sequence consisting of amino acids at positions 19 to 480 or at positions 17 to 480 of the amino acid sequence as shown in SEQ ID NO: 7, or a mixture thereof;
[6] The agent for therapy and/or improvement of disseminated intravascular coagulation according to any one of [1], [2], and [3-4] above, wherein said thrombomodulin is a peptide having a sequence consisting of amino acids at positions 19 to 516 of the amino acid sequence as shown in SEQ ID NO: 9 or 11, a peptide having an amino acid sequence comprising a substitution, deletion or addition of one or multiple amino acids with respect to the amino acid sequence of said peptide and having thrombomodulin activity, or a mixture thereof;
   [6-2] The agent for therapy and/or improvement of disseminated intravascular coagulation according to any one of [1], [2], and [3-4] above, wherein said thrombomodulin is a peptide having a sequence consisting of amino acids at positions 19 to 516 of the amino acid sequence as shown in SEQ ID NO: 9 or 11, or a peptide having an amino acid sequence comprising a substitution, deletion or addition of one or multiple amino acids with respect to the amino acid sequence of said peptide and having thrombomodulin activity;
   [6-3] The agent for therapy and/or improvement of disseminated intravascular coagulation according to any one of [1], [2], and [3-4] above, wherein said thrombomodulin is a peptide having a sequence consisting of amino acids at positions 19 to 516 of the amino acid sequence as shown in SEQ ID NO: 9 or 11;
   [6-4] The agent for therapy and/or improvement of disseminated intravascular coagulation according to any one of [1], [2], and [3-4] above, wherein said thrombomodulin is a peptide having a sequence consisting of amino acids at positions 19 to 516, at positions 19 to 515, at positions 17 to 516, or at positions 17 to 515 of the amino acid sequence as shown in SEQ ID NO: 9 or 11, a peptide having an amino acid sequence comprising a substitution, deletion or addition of one or multiple amino acids with respect to the amino acid sequence of said peptide and having thrombomodulin activity, or a mixture thereof;
   [6-5] The agent for therapy and/or improvement of disseminated intravascular coagulation according to any one of [1], [2], and [3-4] above, wherein said thrombomodulin is a peptide having a sequence consisting of amino acids at positions 19 to 516, at positions 19 to 515, at positions 17 to 516, or at positions 17 to 515 of the amino acid sequence as shown in SEQ ID NO: 9 or 11, or a mixture thereof;
   [6-6] The agent for therapy and/or improvement of disseminated intravascular coagulation according to any one of [1], [2], and [3-4] above, wherein said thrombomodulin is a peptide having a sequence consisting of amino acids at positions 19 to 516, at positions 19 to 515, at positions 17 to 516, or at positions 17 to 515 of the amino acid sequence as shown in SEQ ID NO: 9, or a mixture thereof;
   [6-7] The agent for therapy and/or improvement of disseminated intravascular coagulation according to any one of [1], [2], and [3-4] above, wherein said thrombomodulin is a peptide having a sequence consisting of amino acids at positions 19 to 516, at positions 19 to 515, at positions 17 to 516, or at positions 17 to 515 of the amino acid sequence as shown in SEQ ID NO: 11, or a mixture thereof;
   [6-8] The agent for therapy and/or improvement of disseminated intravascular coagulation according to any one of [1], [2], and [3-4] above, wherein said thrombomodulin is a peptide consisting of a sequence consisting of amino acids at positions 19 to 516, at positions 19 to 515, at positions 17 to 516, or at positions 17 to 515 of the amino acid sequence as shown in SEQ ID NO: 9, or a mixture thereof;
   [6-9] The agent for therapy and/or improvement of disseminated intravascular coagulation according to any one of [1], [2], and [3-4] above, wherein said thrombomodulin is a peptide consisting of a sequence consisting of amino acids at positions 19 to 516, at positions 19 to 515, at positions 17 to 516, or at positions 17 to 515 of the amino acid sequence as shown in SEQ ID NO: 9; [6-10] The agent for therapy and/or improvement of disseminated intravascular coagulation according to any one of [1], [2], and [3-4] above, wherein said thrombomodulin is a peptide consisting of a sequence consisting of amino acids at positions 19 to 516, at positions 19 to 515, at positions 17 to 516, or at positions 17 to 515 of the amino acid sequence as shown in SEQ ID NO: 11, or a mixture thereof;
   [6-11] The agent for therapy and/or improvement of disseminated intravascular coagulation according to any one of [1], [2], and [3-4] above, wherein said thrombomodulin is a peptide consisting of a sequence consisting of amino acids at positions 19 to 516, at positions 19 to 515, at positions 17 to 516, or at positions 17 to 515 of the amino acid sequence as shown in SEQ ID NO: 11;
[7] The agent for therapy and/or improvement of disseminated intravascular coagulation according to any one of [1] to [6-11] above, wherein the patients suffering from disseminated intravascular coagulation have a plasma antithrombin activity of 40% or less.
   It is to be noted that, when there are cited item numbers, such as the aforementioned [1] to [6-11], and when such cited item numbers further include items with hyphenated numbers, such as the aforementioned [6-2], it means that such items with hyphenated numbers are also cited. The same holds true for the below-mentioned items:
[8] The agent for therapy and/or improvement of disseminated intravascular coagulation according to any one of [1] to [7] above, which is a parenteral preparation;
[9] The agent for therapy and/or improvement of disseminated intravascular coagulation according to any one of [1] to [8] above, which is an intravenous preparation;
[10] The agent for therapy and/or improvement of disseminated intravascular coagulation according to any one of [1] to [9] above, which is **characterized in that** it is administered at a dose of 0.02 to 0.08 mg/kg per day within 4 hours via continuous intravenous drip infusion;
[11] The agent for therapy and/or improvement of disseminated intravascular coagulation according to any one of [1] to [10] above, which is **characterized in that** it is not administered in combination with antithrombin;
[12] The agent for therapy and/or improvement of disseminated intravascular coagulation according to any one of [1] to [11] above, wherein the disseminated intravascular coagulation is derived from hemopoietic malignancy or infectious disease.
   [12-1] The agent for therapy and/or improvement of disseminated intravascular coagulation according to any one of [1] to [11] above, wherein the disseminated intravascular coagulation is derived from hemopoietic malignancy.
   [12-2] The agent for therapy and/or improvement of disseminated intravascular coagulation according to any one of [1] to [11] above, wherein the disseminated intravascular coagulation is derived from infectious disease.
   [12-3] An agent for reducing the likelihood of death in human patients suffering from disseminated intravascular coagulation, which comprises thrombomodulin as an active ingredient and which is administered to the patients suffering from disseminated intravascular coagulation who have a plasma antithrombin activity of less than 50%;
   [12-4] The agent according to [12-3] above, wherein the thrombomodulin is the peptide described in any one of [1] to [6-11] above;
   [12-5] The agent according to [12-3] above, which has the characteristics described in any one of [1] to [12-2] above;
[13] A method for selecting patients suffering from disseminated intravascular coagulation, to whom thrombomodulin is to be administered, which comprises: measuring the plasma antithrombin activity of patients suffering from disseminated intravascular coagulation; selecting patients suffering from disseminated intravascular coagulation whose antithrombin activity is less than 50%; and determining that they are patients suffering from disseminated intravascular coagulation, to whom thrombomodulin is to be administered.
   [13-2] The method according to [13] above for selecting patients suffering from disseminated intravascular coagulation, to whom thrombomodulin is to be administered, wherein the thrombomodulin is the peptide described in any one of [1] to [6-11] above;
   [13-3] An agent for therapy and/or improvement of disseminated intravascular coagulation comprising thrombomodulin as an active ingredient, which is administered to patients suffering from disseminated intravascular coagulation after their plasma antithrombin activity has been measured;
   [13-4] An agent for therapy and/or improvement of disseminated intravascular coagulation comprising thrombomodulin as an active ingredient, which is administered to patients suffering from disseminated intravascular coagulation, when their plasma antithrombin activity is measured and the plasma antithrombin activity is less than 50%;
[14] A method for treating and/or improving disseminated intravascular coagulation, which comprises administering thrombomodulin to patients suffering from disseminated intravascular coagulation whose plasma antithrombin activity is less than 50%;
[15] The method according to [14] above, wherein said thrombomodulin is soluble thrombomodulin;
[16] The method according to [14] or [15] above, wherein said thrombomodulin is a peptide obtained from transformant cells prepared by transfecting host cells with DNA encoding the amino acid sequence as shown in SEQ ID NO: 1, 3, 5, 7, 9, or 11;
   [16-2] The method according to [14] or [15] above, wherein said thrombomodulin is a peptide obtained from transformant cells prepared by transfecting host cells with DNA encoding the amino acid sequence as shown in SEQ ID NO: 1 or 3;
   [16-3] The method according to [14] or [15] above, wherein said thrombomodulin is a peptide obtained from transformant cells prepared by transfecting host cells with DNA encoding the amino acid sequence as shown in SEQ ID NO: 5 or 7;
   [16-4] The method according to [14] or [15] above, wherein said thrombomodulin is a peptide obtained from transformant cells prepared by transfecting host cells with DNA encoding the amino acid sequence as shown in SEQ ID NO: 9 or 11;
[17] The method according to any one of [14], [15], and [16-2] above, wherein said thrombomodulin is a peptide having a sequence consisting of amino acids at positions 19 to 132 of the amino acid sequence as shown in SEQ ID NO: 1 or 3, a peptide having a homologous mutation sequence of said sequence and having thrombomodulin activity, or a mixture thereof;
   [17-2] The method according to any one of [14], [15], and [16-2] above, wherein said thrombomodulin is a peptide having a sequence consisting of amino acids at positions 19 to 132 of the amino acid sequence as shown in SEQ ID NO: 1 or 3, or a peptide having a homologous mutation sequence of said sequence and having thrombomodulin activity;
   [17-3] The method according to any one of [14], [15], and [16-2] above, wherein said thrombomodulin is a peptide having a sequence consisting of amino acids at positions 19 to 132 of the amino acid sequence as shown in SEQ ID NO: 1 or 3;
   [17-4] The method according to any one of [14], [15], and [16-2] above, wherein said thrombomodulin is a peptide having a sequence consisting of amino acids at positions 19 to 132 or at positions 17 to 132 of the amino acid sequence as shown in SEQ ID NO: 1 or 3, a peptide having a homologous mutation sequence of said sequence and having thrombomodulin activity, or a mixture thereof;
   [17-5] The method according to any one of [14], [15], and [16-2] above, wherein said thrombomodulin is a peptide having a sequence consisting of amino acids at positions 19 to 132 or at positions 17 to 132 of the amino acid sequence as shown in SEQ ID NO: 1 or 3, or a mixture thereof; [17-6] The method according to any one of [14], [15], and [16-2] above, wherein said thrombomodulin is a peptide having a sequence consisting of amino acids at positions 19 to 132 or at positions 17 to 132 of the amino acid sequence as shown in SEQ ID NO: 1, or a mixture thereof;
   [17-7] The method according to any one of [14], [15], and [16-2] above, wherein said thrombomodulin is a peptide having a sequence consisting of amino acids at positions 19 to 132 or at positions 17 to 132 of the amino acid sequence as shown in SEQ ID NO: 3, or a mixture thereof;
   [17-8] The method according to any one of [14], [15], and [16-2] above, wherein said thrombomodulin is a peptide consisting of a sequence consisting of amino acids at positions 19 to 132 or at positions 17 to 132 of the amino acid sequence as shown in SEQ ID NO: 1, or a mixture thereof;
   [17-9] The method according to any one of [14], [15], and [16-2] above, wherein said thrombomodulin is a peptide consisting of a sequence consisting of amino acids at positions 19 to 132 or at positions 17 to 132 of the amino acid sequence as shown in SEQ ID NO: 3, or a mixture thereof;
[18] The method according to any one of [14], [15], and [16-3] above, wherein said thrombomodulin is a peptide having a sequence consisting of amino acids at positions 19 to 480 of the amino acid sequence as shown in SEQ ID NO: 5 or 7, a peptide having a homologous mutation sequence of said sequence and having thrombomodulin activity, or a mixture thereof;
   [18-2] The method according to any one of [14], [15], and [16-3] above, wherein said thrombomodulin is a peptide having a sequence consisting of amino acids at positions 19 to 480 of the amino acid sequence as shown in SEQ ID NO: 5 or 7, or a peptide having a homologous mutation sequence of said sequence and having thrombomodulin activity;
   [18-3] The method according to any one of [14], [15], and [16-3] above, wherein said thrombomodulin is a peptide having a sequence consisting of amino acids at positions 19 to 480 of the amino acid sequence as shown in SEQ ID NO: 5 or 7;
   [18-4] The method according to any one of [14], [15], and [16-3] above, wherein said thrombomodulin is a peptide having a sequence consisting of amino acids at positions 19 to 480 or at positions 17 to 480 of the amino acid sequence as shown in SEQ ID NO: 5 or 7, a peptide having a homologous mutation sequence of said sequence and having thrombomodulin activity, or a mixture thereof;
   [18-5] The method according to any one of [14], [15], and [16-3] above, wherein said thrombomodulin is a peptide having a sequence consisting of amino acids at positions 19 to 480 or at positions 17 to 480 of the amino acid sequence as shown in SEQ ID NO: 5 or 7, or a mixture thereof;
   [18-6] The method according to any one of [14], [15], and [16-3] above, wherein said thrombomodulin is a peptide having a sequence consisting of amino acids at positions 19 to 480 or at positions 17 to 480 of the amino acid sequence as shown in SEQ ID NO: 5, or a mixture thereof;
   [18-7] The method according to any one of [14], [15], and [16-3] above, wherein said thrombomodulin is a peptide having a sequence consisting of amino acids at positions 19 to 480 or at positions 17 to 480 of the amino acid sequence as shown in SEQ ID NO: 7, or a mixture thereof;
   [18-8] The method according to any one of [14], [15], and [16-3] above, wherein said thrombomodulin is a peptide consisting of a sequence consisting of amino acids at positions 19 to 480 or at positions 17 to 480 of the amino acid sequence as shown in SEQ ID NO: 5, or a mixture thereof;
   [18-9] The method according to any one of [14], [15], and [16-3] above, wherein said thrombomodulin is a peptide consisting of a sequence consisting of amino acids at positions 19 to 480 or at positions 17 to 480 of the amino acid sequence as shown in SEQ ID NO: 5, or a mixture thereof;
[19] The method according to any one of [14], [15], and [16-4] above, wherein said thrombomodulin is a peptide having a sequence consisting of amino acids at positions 19 to 516 of the amino acid sequence as shown in SEQ ID NO: 9 or 11, a peptide having a homologous mutation sequence of said sequence and having thrombomodulin activity, or a mixture thereof;
   [19-2] The method according to any one of [14], [15], and [16-4] above, wherein said thrombomodulin is a peptide having a sequence consisting of amino acids at positions 19 to 516 of the amino acid sequence as shown in SEQ ID NO: 9 or 11, or a peptide having a homologous mutation sequence of said sequence and having thrombomodulin activity;
   [19-3] The method according to any one of [14], [15], and [16-4] above, wherein said thrombomodulin is a peptide having a sequence consisting of amino acids at positions 19 to 516 of the amino acid sequence as shown in SEQ ID NO: 9 or 11;
   [19-4] The method according to any one of [14], [15], and [16-4] above, wherein said thrombomodulin is a peptide having a sequence consisting of amino acids at positions 19 to 516, at positions 19 to 515, at positions 17 to 516, or at positions 17 to 515 of the amino acid sequence as shown in SEQ ID NO: 9 or 11, a peptide having a homologous mutation sequence of said sequence and having thrombomodulin activity, or a mixture thereof;
   [19-5] The method according to any one of [14], [15], and [16-4] above, wherein said thrombomodulin is a peptide having a sequence consisting of amino acids at positions 19 to 516, at positions 19 to 515, at positions 17 to 516, or at positions 17 to 515 of the amino acid sequence as shown in SEQ ID NO: 9 or 11, or a mixture thereof;
   [19-6] The method according to any one of [14], [15], and [16-4] above, wherein said thrombomodulin is a peptide having a sequence consisting of amino acids at positions 19 to 516, at positions 19 to 515, at positions 17 to 516, or at positions 17 to 515 of the amino acid sequence as shown in SEQ ID NO: 9, or a mixture thereof;
   [19-7] The method according to any one of [14], [15], and [16-4] above, wherein said thrombomodulin is a peptide having a sequence consisting of amino acids at positions 19 to 516, at positions 19 to 515, at positions 17 to 516, or at positions 17 to 515 of the amino acid sequence as shown in SEQ ID NO: 11, or a mixture thereof;
   [19-8] The method according to any one of [14], [15], and [16-4] above, wherein said thrombomodulin is a peptide consisting of a sequence consisting of amino acids at positions 19 to 516, at positions 19 to 515, at positions 17 to 516, or at positions 17 to 515 of the amino acid sequence as shown in SEQ ID NO: 9, or a mixture thereof;
   [19-9] The method according to any one of [14], [15], and [16-4] above, wherein said thrombomodulin is a peptide consisting of a sequence consisting of amino acids at positions 19 to 516, at positions 19 to 515, at positions 17 to 516, or at positions 17 to 515 of the amino acid sequence as shown in SEQ ID NO: 9;
   [19-10] The method according to any one of [14], [15], and [16-4] above, wherein said thrombomodulin is a peptide consisting of a sequence consisting of amino acids at positions 19 to 516, at positions 19 to 515, at positions 17 to 516, or at positions 17 to 515 of the amino acid sequence as shown in SEQ ID NO: 11, or a mixture thereof;
   [19-11] The method according to any one of [14], [15], and [16-4] above, wherein said thrombomodulin is a peptide consisting of a sequence consisting of amino acids at positions 19 to 516, at positions 19 to 515, at positions 17 to 516, or at positions 17 to 515 of the amino acid sequence as shown in SEQ ID NO: 11;
[20] The method according to any one of [14] to [19-11] above, which comprises administering thrombomodulin to the patients suffering from disseminated intravascular coagulation who have a plasma antithrombin activity of 40% or less;
[21] The method according to any one of [14] to [20] above, wherein thrombomodulin is administered parenterally;
[22] The method according to any one of [14] to [21] above, wherein thrombomodulin is administered intravenously;
[23] The method according to any one of [14] to [22] above, wherein thrombomodulin is administered at a dose of 0.02 to 0.08 mg/kg per day within 4 hours via continuous intravenous drip infusion;
[24] The method according to any one of [14] to [23] above, which is **characterized in that** thrombomodulin is administered without combining with antithrombin;
[25] The method according to any one of [14] to [24] above, wherein the disseminated intravascular coagulation is derived from hemopoietic malignancy or infectious disease.
   [25-1] The method according to any one of [14] to [24] above, wherein the disseminated intravascular coagulation is derived from hemopoietic malignancy.
   [25-2] The method according to any one of [14] to [24] above, wherein the disseminated intravascular coagulation is derived from infectious disease.
[26] A method for reducing the likelihood of death in human patients suffering from disseminated intravascular coagulation, which comprises administering thrombomodulin to the patients suffering from disseminated intravascular coagulation who have a plasma antithrombin activity of less than 50%;
   [26-2] The method according to [26] above, wherein the thrombomodulin is the peptide described in any one of [1] to [6-11] above;
   [26-3] The method according to [26] above, which has the characteristics described in any one of [1] to [25-2] above;
[27] A method for treating and/or improving disseminated intravascular coagulation, which comprises administering thrombomodulin to patients suffering from disseminated intravascular coagulation after their plasma antithrombin activity has been measured;
   [27-2] A method for treating and/or improving disseminated intravascular coagulation, which comprises administering thrombomodulin to patients suffering from disseminated intravascular coagulation, when their plasma antithrombin activity is measured and the plasma antithrombin activity is less than 50%.

### Effects of the Invention

By the use of the thrombomodulin-containing preparation of the present invention, the symptoms of DIC patients having a plasma AT activity of less than 50% can be effectively treated and/or improved, preferably without combining with AT.

### BEST MODE FOR CARRYING OUT THE INVENTION

The present invention will be specifically described below.

The present invention provides an agent for therapy and/or improvement of disseminated intravascular coagulation (DIC) comprising thrombomodulin as an active ingredient, which is administered to patients suffering from the disseminated intravascular coagulation who have a plasma antithrombin activity of less than 50%.

That is, the agent for therapy and/or improvement of disseminated intravascular coagulation of the present invention is an agent for therapy and/or improvement of disseminated intravascular coagulation in patients suffering from the disseminated intravascular coagulation who have a plasma antithrombin activity of less than 50%.

Moreover, the present invention also provides an agent for reducing the likelihood of death in human patients suffering from disseminated intravascular coagulation.

A preferred effect obtained from the therapy and/or improvement of DIC is "prevention of the death of patients due to DIC" for example. In addition, another preferred effect is "prevention of deterioration of the systemic condition of patients due to DIC".

DIC is a disease or syndrome whereby large quantities of blood coagulation-accelerating substances are generated as a result of tissue damage caused by various diseases, so that the function of a coagulation system is excessively accelerated, and small thrombuses are formed in generalized blood vessel (microthrombus formation) and they clog small vessels, and at the same time, thrombocytes or coagulation factors necessary for the control of bleeding are consumed, thereby causing clotting abnormality. Specifically, as a result of fibrin formation in vascular vessel, bleeding due to consumption coagulopathy or organ failure due to microthrombus formation occurs. DIC is also referred to as disseminated intravascular coagulation syndrome or diffuse intravascular coagulation syndrome.

In the case of underlying disease that is malignant tumor such as hemopoietic malignancy or solid carcinoma, when a tissue factor expressed in tumor cells is allowed to come into contact with blood, a blood coagulation system is excessively activated to cause DIC.

Moreover, DIC may also be caused by systemic inflammatory response syndrome (hereinafter abbreviated as SIRS, at times) that occurs as a result of severe, infectious or noninfectious, clinical invasion.

SIRS is a state caused by accelerated generation of inflammatory cytokine attended with serious tissue injury or severe infectious disease. If this reaction becomes excessive, DIC is caused by activation of neutrophil or activation of vascular endothelial cells. That is to say, this state is a preliminary stage of DIC (hereinafter abbreviated as preDIC, at times), and the agent for therapy and/or improvement of DIC of the present invention can be effectively used at preDIC. Thus, the intended use of the agent for therapy and/or improvement of DIC of the present invention also includes the therapy and/or improvement of preDIC.

DIC has various types of clinical symptoms depending on the type of underlying pathogenic condition. In addition to observation of bleeding or organ symptoms, a preferred method for diagnosing an illness as DIC comprises keeping the score of DIC on the basis of several test values as described below and then diagnosing the illness as DIC when the DIC score has reached a certain level. Examples of such test values include the number of blood platelets, the concentration of fibrin/fibrinogen degradation products (hereinafter abbreviated as FDP, at times) decomposed by plasmin, a D-dimer concentration, a fibrinogen concentration, and a prothrombin time. Moreover, it is also possible to diagnose a certain condition as preDIC based on a decrease in platelets, an increase in the D-dimer or FDP concentration, etc. (Masao Nakagawa, "Search report regarding use of criteria of disseminated intravascular coagulation (DIC)," Research Study Team of Intractable Disease (Blood Coagulation Abnormality), the Ministry of Health and Welfare, Study report 1999, 1999: 65-72; Katsumi Deguchi, "Tentative plan regarding standards for initiation of early treatment of DIC," Research Study Team of Intractable Disease (Blood Coagulation Abnormality), the Ministry of Health and Welfare, Study report 1999, 1999: 73-77; and Katsumi Nakagawa & Hajime Tsuji, "Current diagnosis of DIC - Reports on results of inquiry survey" Clinical Blood. 1999, 40: 362-364). Furthermore, it is also possible to diagnose a certain condition as preDIC by measuring a soluble fibrin concentration in blood or a thrombin-antithrombin complex concentration in blood. Specific examples of DIC scoring criteria include Overt DIC criteria (Taylor FB et al., Thromb Haemost 2001: 86: 1327-1330), acute-phase DIC criteria (Gando S. et al., Clin Appl Thromb Hemost 2005: 11(1): 71-76), and the DIC criteria of the Ministry of Health and Welfare (Nobuo Aoki & Jun Hasegawa, "Revision of the chapter 'Auxiliary test results and findings for the diagnosis' in DIC criteria," Research Study Team of Intractable Disease (Blood Coagulation Abnormality), the Ministry of Health and Welfare, Study report 1992, 1988: pp. 37-41).

A method for diagnosing an illness as DIC is not particularly limited, as long as it is the aforementioned diagnostic method. A diagnostic method comprising keeping DIC score is preferable. In addition, from the viewpoint of therapeutic effects, the cost of medical care, and the QOL of patients, if an early treatment for DIC is necessary, there is another embodiment in which preDIC diagnosis is preferably carried out.

As diagnostic methods comprising keeping DIC score, there is another diagnostic method comprising keeping similar DIC score, as well as the aforementioned diagnostic method.

The type of DIC, to which the agent for therapy and/or improvement of DIC of the present invention is to be administered, is not particularly limited. However, the agent of the present invention is applied more preferably to hemopoietic malignancy-derived DIC or infectious disease-derived DIC, and further preferably to infectious disease-derived DIC. A specific example of the infectious disease-derived DIC is preferably septicemia-derived DIC. In another preferred embodiment, the agent of the present invention is also applied to hemopoietic malignancy-derived DIC.

The agent for therapy and/or improvement of DIC of the present invention may also be used for septicemia. Septicemia may also be considered to be SIRS occurring as a result of severe, infectious, clinical invasion of infectious disease. Thus, septicemia is closely associated with DIC caused by infectious disease. Septicemia often occurs with DIC. There may be cases where the agent of the present invention can be used for patients with both septicemia and DIC. That is, in the present invention, the agent may be used for patients with either septicemia or DIC, or patients with both septicemia and DIC, or patients suspected to have septicemia and/or DIC.

Septicemia has been known as a serious systemic infectious disease that occurs due to continuous or intermittent infiltration of microorganisms from an infectious focus into blood, as a result of diseases such as infectious disease, malignant tumor, hepatocirrhosis, renal failure, diabetes or dystocia, or treatments for injury or diseases, such as indwelling catheters, transfusion solution devices, dialysis or tracheostomy. If the symptoms of this disease progress, systemic shock is induced by septic shock, namely, by a sharp fall in blood pressure or peripheral circulatory failure. Thereafter, the occurrence of disorder in important organs such as lung, kidney, liver, heart, alimentary canal and central nerve system leads to death. Moreover, as complications of septicemia, there is induced DIC or adult respiratory distress syndrome (ARDS) characterized by pulmonary interstitial edema, bleeding or acute respiratory failure caused by pulmonary capillary disorder attended with activation of neutrophil and the migration and accumulation thereof in lung parenchyma. The prognosis is extremely poor.

There are several methods for diagnosing an illness as septicemia. Such methods are summarized in Levy M. et al., Crit Care. Med., 31: 1250-1256. Examples of the septicemia diagnostic methods include methods for diagnosing an illness as septicemia by doctors and methods using test values and the like. An example of the latter methods is a method for diagnosing an illness as septicemia, which comprises diagnosing the illness as SIRS when two out of the following four items ((1) a body temperature of >38°C or <36°C, (2) a cardiac rate of >90/min, (3) a respiration rate of >20/min or artificial respiration required, and (4) a leukocyte count of >12,000/mm³ or < 4,000/mm³, or an immature leukocyte count of >10%) have been satisfied, and then diagnosing the SIRS as septicemia when the cause of the disease has been demonstrated or suspected to be a microorganism [LaRosa S., the homepage of the Cleveland Clinic]. A method similar to this method is described in Members of the American College of Chest Physicians/Society of Critical Care Medicine Consensus Conference: Crit Care Med, 20, 864-874 (1992).

Examples of the conditions of septicemia patients include bacteremia, septicemia, systemic inflammatory response syndrome (SIRS), septicemia (which is SIRS in which the cause of the disease has been demonstrated or suspected to be a microorganism), severe septicemia, septic shock, intractable septic shock, and multiple organ dysfunction syndrome (hereinafter referred to as MODS, at times) (Harrison's Principles of Internal Medicine, original article 15th edition, 124: pp. 828-833, Medical Science International). Each of the aforementioned conditions is exemplified as a symptom, for which the therapeutic and/or improving agent of the present invention is effectively used.

As bacteremia, a condition in which the presence of bacteria in blood is confirmed by positive blood culture is exemplified.

As septicemia, a condition in which the presence of a microorganism or other toxins in blood is confirmed is exemplified.

As systemic inflammatory response syndrome (SIRS), a preliminary stage of DIC is exemplified, as described above.

As severe septicemia, septicemia accompanied with one or multiple symptoms selected from organ failure and hypotension, such as metabolic acidosis, acute encephalopathy, oliguria, hypoxemia and disseminated intravascular coagulation, is exemplified.

As septic shock, a condition which involves low blood pressure (a blood pressure of 90 mmHg or less, or a blood pressure that is 40 mmHg or less lower than ordinary blood pressure), which does not react with reanimation using infusion solution, and which is accompanied with organ failure, is exemplified.

As intractable septic shock, septic shock that continues for 1 hour or more and does not react with a vasopressor contained in infusion solution is exemplified.

As multiple organ dysfunction syndrome (MODS), a disease that is accompanied with failure of one or multiple organs and requires medical intervention to maintain homeostasis is exemplified.

The agent for therapy and/or improvement of DIC of the present invention can be effectively used for patients with low AT activity. The term "patients with low AT activity" is used to mean patients whose plasma AT activity is lower than that of normal persons (70% or less). As such patients with low AT activity, congenital AT-deficient patients and acquired AT-deficient patients are exemplified. The congenital AT-deficient patients mean patients whose AT activity is naturally lower than that of normal persons (approximately 35% to 70%). On the other hand, acquired AT-deficient patients mean patients whose AT activity is decreased to 70% or less of that of normal persons after birth. Specific causes for decreasing AT activity after birth include liver diseases such as chronic liver failure, postoperative conditions, shock, severe infectious disease, renal diseases such as nephrotic syndrome, digestive system diseases such as inflammatory bowel disease, a decrease in AT activity due to heparin or the like, a decrease in AT activity due to aging, premature birth newborn, diabetes, Behcet's disease, and malnutrition. In the present invention, however, whether the DIC disease of patients with low AT activity is infected congenitally or the disease is acquired after birth is not particularly limited. The race of such patients is not particularly limited. Japanese is preferable.

Among others, the DIC symptoms of patients with a plasma AT activity of less than 50% can be effectively treated and/or improved using the agent for therapy and/or improvement of DIC of the present invention comprising thrombomodulin as an active ingredient.

AT activity is defined as activity of neutralizing the coagulant activity of thrombin or activated factor X. In general, the AT activity is indicated as a relative value to the AT activity obtained from the plasma of a healthy adult (hereinafter referred to as normal plasma). The type of the normal plasma used is not particularly limited, as long as it is normal plasma that is commonly used by persons skilled in the art. There can be used international standard products of normal plasma (manufactured by NIBSC), which have been established by the Expert Committee on Biological Standardization (hereinafter abbreviated as ECBS, at times) of the World Health Organization (WHO) and have been then controlled and distributed by the National Institute for Biological Standards and Control (hereinafter abbreviated as NIBSC, at times). Moreover, as another preferred example, there can also be used commercially available standard plasma whose AT activity has been corrected using the international standard products of NIBSC (for example, standard plasma of the Scientific and Standardization Committee (hereinafter abbreviated as SSC, at times) and the International Society on Thrombosis and Haemostasis (hereinafter abbreviated as ISTH, at times)). Furthermore, normal plasma included with a common AT activity measurement kit or normal plasma designated by such an AT activity measurement kit can also be used. As such normal plasma included with a common AT activity measurement kit or normal plasma designated by the AT activity measurement kit, a normal plasma preparation "Daiichi" (Daiichi Kagaku Co., Ltd.) is preferably used. In some cases, the AT activity of a plasma sample measured using such normal plasma may preferably be converted to the AT activity obtained using, as a standard, the normal plasma that is the international standard product of NIBSC. More preferably, the AT activity can be obtained by converting it to the AT activity obtained using the normal plasma preparation "Daiichi" (Daiichi Kagaku Co., Ltd.) as a standard.

As normal plasma, the international standard product of NIBSC is preferably used. In another embodiment, commercially available standard plasma whose AT activity has been corrected using the international standard product of NIBSC is preferably used. In a more preferred embodiment, the normal plasma preparation "Daiichi" (Daiichi Kagaku Co., Ltd.) is used as such normal plasma. For example, when the AT activity in plasma is 50%, it means that the ratio of the AT activity in the plasma to the AT activity in the aforementioned normal plasma is 50%.

In order to effectively treat and/or improve the symptoms of DIC, the plasma AT activity of DIC patients is not particularly limited, as long as it is less than 50%. The upper limit of the plasma AT activity is preferably 48% or less, more preferably 46% or less, further preferably 44% or less, particularly preferably 42% or less, and most preferably 40% or less. On the other hand, the lower limit of the plasma AT activity is not particularly limited, as long as it is equal to or higher than the detection limit of the aforementioned measurement method. It is, for example, 0.1% or more, preferably 10% or more, more preferably 15% or more, further preferably 20% or more, and particularly preferably 25% or more. Taking into consideration the AT activity of the patient group in Test example 1 of the example section, the lower limit of the plasma AT activity is most preferably 30% or more.

Moreover, the present invention provides an agent for therapy and/or improvement of disseminated intravascular coagulation comprising thrombomodulin as an active ingredient, which is administered to patients suffering from the disseminated intravascular coagulation after their plasma antithrombin activity has been measured. The type of the agent for therapy and/or improvement of disseminated intravascular coagulation of the present invention is not particularly limited, as long as it is an agent that is administered after the plasma antithrombin activity of the patients has been measured. It is preferably an agent for therapy and/or improvement of disseminated intravascular coagulation that is administered to patients suffering from the disseminated intravascular coagulation, when the measured antithrombin activity is less than 50%. Thus, administration of thrombomodulin to patient suffering from disseminated intravascular coagulation after their antithrombin activity has been measured is preferable for allowing the agent to sufficiently exhibit its effects.

Furthermore, it is also possible to administer the agent for therapy and/or improvement of DIC of the present invention to patients having low AT activity that has been decreased due to liver diseases such as chronic liver failure, postoperative conditions, shock, severe infectious disease, etc.

The thrombomodulin used in the present invention has been known to have action to (1) selectively bind to thrombin and to (2) promote the activation of protein C caused by the thrombin. In addition, it is preferable that the present thrombomodulin generally have (3) action to extend the coagulation time by thrombin and/or (4) action to suppress the platelet aggregation caused by thrombin. Such action of thrombomodulin may be referred to as thrombomodulin activity.

As such thrombomodulin activity, thrombomodulin preferably has the actions in (1) and (2) above, and more preferably has all the actions in (1) to (4) above.

As the action to promote the activation of protein C caused by thrombin, the activity level of the action to promote the activation of protein C or the presence of absence of such action can easily be confirmed by applying the test methods clearly described in various types of known publications including JP Patent Publication (Kokai) No. 64-6219 A (1989). Moreover, the action to extent the coagulation time caused by thrombin or the action to suppress the platelet aggregation caused by thrombin can also be confirmed in the same above manner.

The type of the thrombomodulin used in the present invention is not particularly limited, as long as it has thrombomodulin activity. It is preferably soluble thrombomodulin. As preferred examples of the solubility of such soluble thrombomodulin, it is dissolved in water such as distilled water used for injection (in the absence of a surfactant such as Triton X-100 or polidocanol, and generally around the neutral range) at an amount of 1 mg/mL or more or 10 mg/mL or more; preferably 15 mg/mL or more or 17 mg/mL or more; more preferably 20 mg/mL or more, 25 mg/mL or more, or 30 mg/mL or more; and particularly preferably 60 mg/mL or more. In some cases, such soluble thrombomodulin is dissolved in water at an amount of 80 mg/ML or more or 100 mg/mL or more. In order to clearly determine whether or not soluble thrombomodulin has been dissolved in water, after it has been dissolved in water, the water is observed by naked eye immediately under white light at a position of illumination of approximately 1000, for example. If the water looks clear and does not contain insoluble substances to such an extent that they are clearly observed, it can be a clear indicator for the soluble thrombomodulin that has been dissolved in the water. It is also possible to confirm it by filtrating the water and examining the presence or absence of residue.

The thrombomodulin used in the present invention preferably comprises an amino acid sequence consisting of amino acids at positions 19 to 132 of the amino acid sequence as shown in SEQ ID NO: 1, which has been known as the central portion of the thrombomodulin activity of human thrombomodulin. The amino acid sequence of the present thrombomodulin is not particularly limited, as long as it comprises an amino acid sequence consisting of amino acids at positions 19 to 132 of the amino acid sequence as shown in SEQ ID NO: 1. The amino acid sequence consisting of amino acids at positions 19 to 132 of the above-described amino acid sequence as shown in SEQ ID NO: 1 may be naturally or artificially mutated, as long as it has action to promote the activation of protein C caused by thrombin, namely, thrombomodulin activity. That is to say, the amino acid sequence consisting of amino acids at positions 19 to 132 may comprise a substitution, deletion, or addition of one or multiple amino acids with respect to the amino acid sequence as shown in SEQ ID NO: 1. The acceptable level of mutation is not particularly limited, as long as the aforementioned amino acid sequence has thrombomodulin activity. The mutated amino acid sequence consisting of amino acids at positions 19 to 132 of the amino acid sequence as shown in SEQ ID NO: 1 shows homology of, for example, 50% or more, preferably 70% or more, more preferably 80% or more, further preferably 90% or more, particularly preferably 95% or more, and most preferably 98% or more, with the original amino acid sequence. Such an amino acid sequence is referred to as a homologous mutation sequence. As described later, such a mutated amino acid sequence can be easily produced by a common genetic engineering technique.

In the amino acid sequence as shown in SEQ ID NO: 3, Val that is the amino acid at position 125 of the amino acid sequence as shown in SEQ ID NO: 1 has been substituted with Ala. The thrombomodulin used in the present invention also preferably includes an amino acid sequence consisting of amino acids at positions 19 to 132 of the amino acid sequence as shown in SEQ ID NO: 3.

Thus, the type of the thrombomodulin used in the present invention is not particularly limited, as long as it has, at least, an amino acid sequence consisting of amino acids at positions 19 to 132 of the amino acid sequence as shown in SEQ ID NO: 1 or 3, or a homologous mutation sequence of the aforementioned sequence, and it comprises at least a peptide sequence having thrombomodulin activity. Preferred examples of the present thrombomodulin include a peptide consisting of a sequence consisting of amino acids at positions 19 to 132 or at positions 17 to 132 of the amino acid sequence as shown in SEQ ID NO: 1 or 3, and a peptide consisting of a homologous mutation sequence of the aforementioned sequence and having at least thrombomodulin activity. A peptide consisting of a sequence consisting of amino acids at positions 19 to 132 of the amino acid sequence as shown in SEQ ID NO: 1 or 3 is more preferable. Moreover, in another more preferred embodiment, there can also be applied a peptide consisting of a homologous mutation sequence of the amino acid sequence consisting of amino acids at positions 19 to 132 or at positions 17 to 132 of the amino acid sequence as shown in SEQ ID NO: 1 or 3 and having at least thrombomodulin activity.

In another embodiment, the thrombomodulin used in the present invention preferably comprises an amino acid sequence consisting of amino acids at positions 19 to 480 of the amino acid sequence as shown in SEQ ID NO: 5. The type of the present thrombomodulin in this embodiment is not particularly limited, as long as it comprises such an amino acid sequence consisting of amino acids at positions 19 to 480 of the amino acid sequence as shown in SEQ ID NO: 5. The amino acid sequence consisting of amino acids at positions 19 to 480 of the aforementioned amino acid sequence as shown in SEQ ID NO: 5 may be homologously mutated, as long as it has action to promote the activation of protein C caused by thrombin, namely, thrombomodulin activity.

In the amino acid sequence as shown in SEQ ID NO: 7, Val that is the amino acid at position 473 of the amino acid sequence as shown in SEQ ID NO: 5 has been substituted with Ala. The thrombomodulin used in the present invention also preferably includes an amino acid sequence consisting of amino acids at positions 19 to 480 of the amino acid sequence as shown in SEQ ID NO: 7.

Thus, the type of the thrombomodulin used in the present invention is not particularly limited, as long as it has, at least, an amino acid sequence consisting of amino acids at positions 19 to 480 of the amino acid sequence as shown in SEQ ID NO: 5 or 7, or a homologous mutation sequence of the aforementioned sequence, and it comprises at least a peptide sequence having thrombomodulin activity. Preferred examples of the present thrombomodulin include a peptide consisting of a sequence consisting of amino acids at positions 19 to 480 or at positions 17 to 480 of the amino acid sequence as shown in SEQ ID NO: 5 or 7, and a peptide consisting of a homologous mutation sequence of the aforementioned sequence and having at least thrombomodulin activity. A peptide consisting of a sequence consisting of amino acids at positions 19 to 480 of the amino acid sequence as shown in SEQ ID NO: 5 or 7 is more preferable. Moreover, in a further more preferred embodiment, there can also be applied a peptide consisting of a homologous mutation sequence of the amino acid sequence consisting of amino acids at positions 19 to 480 or at positions 17 to 480 of the amino acid sequence as shown in SEQ ID NO: 5 or 7 and having at least thrombomodulin activity.

In a further embodiment, the thrombomodulin used in the present invention preferably includes an amino acid sequence consisting of amino acids at positions 19 to 515 of the amino acid sequence as shown in SEQ ID NO: 9. The type of the present thrombomodulin in this embodiment is not particularly limited, as long as it comprises such an amino acid sequence consisting of amino acids at positions 19 to 515 of the amino acid sequence as shown in SEQ ID NO: 9. The amino acid sequence consisting of amino acids at positions 19 to 515 of the aforementioned amino acid sequence as shown in SEQ ID NO: 9 may be homologously mutated, as long as it has action to promote the activation of protein C caused by thrombin, namely, thrombomodulin activity.

In the amino acid sequence as shown in SEQ ID NO: 11, Val that is the amino acid at position 473 of the amino acid sequence as shown in SEQ ID NO: 9 has been substituted with Ala. The thrombomodulin used in the present invention also preferably includes an amino acid sequence consisting of amino acids at positions 19 to 515 of the amino acid sequence as shown in SEQ ID NO: 11.

Thus, the type of the thrombomodulin used in the present invention is not particularly limited, as long as it has, at least, an amino acid sequence consisting of amino acids at positions 19 to 515 of the amino acid sequence as shown in SEQ ID NO: 9 or 11, or peptide sequence having a homologous mutation sequence of the aforementioned sequence and having at least thrombomodulin activity. More preferred examples of the present thrombomodulin include a peptide consisting of a sequence consisting of amino acids at positions 19 to 516, at positions 19 to 515, at positions 17 to 516, or at positions 17 to 515 of the amino acid sequence as shown in SEQ ID NO: 9 or 11, and a peptide consisting of a homologous mutation sequence of the aforementioned sequence and having at least thrombomodulin activity. A peptide consisting of a sequence consisting of amino acids at positions 19 to 516, at positions 19 to 515, at positions 17 to 516, or at positions 17 to 515 of the amino acid sequence as shown in SEQ ID NO: 9 is particularly preferable. In addition, a mixture thereof is also a preferred example. Moreover, in another particularly preferred embodiment, there can also be applied a peptide consisting of a sequence consisting of amino acids at positions 19 to 516, at positions 19 to 515, at positions 17 to 516, or at positions 17 to 515 of the amino acid sequence as shown in SEQ ID NO: 11. A mixture thereof is also a preferred example of the thrombomodulin of the present invention. Further, a peptide consisting of a homologous mutation sequence thereof and having at least thrombomodulin activity is also another preferred example of the present thrombomodulin.

A peptide having a homologous mutation sequence is as described above. Such a peptide having a homologous mutation sequence also includes a peptide that may comprise a substitution, deletion, or addition of one or more, namely; one or multiple, and preferably several (for example, 1 to 20, preferably 1 to 10, more preferably 1 to 5, and particularly preferably 1 to 3) amino acids in the amino acid sequence of the target peptide. The acceptable level of mutation is not particularly limited, as long as the peptide has thrombomodulin activity. The mutated peptide shows homology of, for example, 50% or more, preferably 70% or more, more preferably 80% or more, further preferably 90% or more, particularly preferably 95% or more, and most preferably 98% or more, at the amino acid sequence level with the target peptide.

Moreover, other preferred examples of the thrombomodulin used in the present invention include a peptide consisting of a sequence (462 amino acid residues) as shown in SEQ ID NO: 14, a peptide consisting of a sequence (272 amino acid residues) as shown in SEQ ID NO: 8, and a peptide consisting of a sequence (236 amino acid residues) as shown in SEQ ID NO: 6, which are described in JP Patent Publication (Kokai) No. 64-6219 (1989) A.

The type of the thrombomodulin used in the present invention is not particularly limited, as long as it has at least an amino acid sequence consisting of amino acids at positions 19 to 132 of the amino acid sequence as shown in SEQ ID NO: 1 or 3. Among others, a peptide having at least an amino acid sequence consisting of amino acids at positions 19 to 480 of the amino acid sequence as shown in SEQ ID NO: 5 or 7 is preferable, and a peptide having at least an amino acid sequence consisting of amino acids at positions 19 to 515 of the amino acid sequence as shown in SEQ ID NO: 9 or 11 is more preferable. A more preferred example of the peptide having at least an amino acid sequence consisting of amino acids at positions 19 to 515 of the amino acid sequence as shown in SEQ ID NO: 9 or 11 is a peptide consisting of a sequence consisting of amino acids at positions 19 to 516, at positions 19 to 515, at positions 17 to 516, or at positions 17 to 515 of the amino acid sequence as shown in SEQ ID NO: 9 or 11. Furthermore, a mixture obtained from such peptides each consisting of a sequence consisting of amino acids at positions 19 to 516, at positions 19 to 515, at positions 17 to 516, or at positions 17 to 515 of the amino acid sequence as shown in SEQ ID NO: 9 or 11 is also a preferred example of the thrombomodulin of the present invention.

In the case of the aforementioned mixture, the mixing ratio between a peptide that starts from position 17 of the amino acid sequence as shown in SEQ ID NO: 9 or 11 and a peptide that starts from position 19 thereof is (30 : 70) to (50 : 50), and preferably (35 : 65) to (45 : 55).

Moreover, the mixing ratio between a peptide that terminates at position 515 of the amino acid sequence as shown in SEQ ID NO: 9 or 11 and a peptide that terminates at position 516 is (70 : 30) to (90 : 10), and preferably (75 : 25) to (85 : 15).

The mixing ratio of such peptides can be obtained by a common method.

It is to be noted that the sequence consisting of amino acids at positions 19 to 132 of the amino acid sequence as shown in SEQ ID NO: 1 corresponds to the sequence consisting of amino acids at positions 367 to 480 of the amino acid sequence as shown in SEQ ID NO: 9, and that the sequence consisting of amino acids at positions 19 to 480 of the amino acid sequence as shown in SEQ ID NO: 5 corresponds to the sequence consisting of amino acids at positions 19 to 480 of the amino acid sequence as shown in SEQ ID NO: 9.

Further, the sequence consisting of amino acids at positions 19 to 132 of the amino acid sequence as shown in SEQ ID NO: 3 corresponds to the sequence consisting of amino acids at positions 367 to 480 of the amino acid sequence as shown in SEQ ID NO: 11, and the sequence consisting of amino acids at positions 19 to 480 of the amino acid sequence as shown in SEQ ID NO: 7 corresponds to the sequence consisting of amino acids at positions 19 to 480 of the amino acid sequence as shown in SEQ ID NO: 11.

Still further, the sequences each consisting of amino acids at positions 1 to 18 of the amino acid sequences as shown in SEQ ID NOS: 1, 3, 5, 7, 9, and 11 are all identical to one another.

As described below, the thrombomodulin used in the present invention can be obtained from transformant cells that are prepared by incorporating DNA encoding the peptide having the amino acid sequence as shown in SEQ ID NO: 1, 3, 5, 7, 9, 11, etc. (specifically, the nucleotide sequence as shown in SEQ ID 2, 4, 6, 8, 10, 12, etc., respectively) into a vector and then by transfecting host cells with the vector.

It is only necessary that such peptides have the aforementioned amino acid sequences. Thus, a sugar chain may be or may not be added. Addition of a sugar chain is not particularly limited. In addition, in genetic engineering techniques, the type of such a sugar chain, a position to which a sugar chain is added, and the level of addition differ depending on the type of host cell used, and they are not particularly limited. The binding position of such a sugar chain and the type thereof are described in JP Patent Publication (Kokai) No. 11-341990 (1999) A. In the case of the thrombomodulin of the present invention, the same sugar chain may be added to the same position as those described in the aforementioned publication. As described later, the method for obtaining the thrombomodulin of the present invention is not limited to genetic engineering. In the case of obtaining the present thrombomodulin by genetic engineering, however, as a signal sequence that can be used in expression, a nucleotide sequence encoding the amino acid sequence consisting of amino acids at positions 1 to 18 of the aforementioned amino acid sequence as shown in SEQ ID NO: 9, a nucleotide sequence encoding the amino acid sequence consisting of amino acids at positions 1 to 16 of the aforementioned amino acid sequence as shown in SEQ ID NO: 9, and other known signal sequences such as the signal sequence of a human tissue plasminogen activator can be used (International Publication WO88/9811, and JP Patent Publication (Kokai) No. 11-341990 (1999) A).

When a DNA sequence encoding thrombomodulin is introduced into host cells, there is preferably applied a method, which comprises incorporating the DNA sequence encoding thrombomodulin into a vector, and more preferably into an expression vector capable of expressing in animal cells, and then introducing the vector into the host cells. Such an expression vector is a DNA molecule that is constituted with a promoter sequence, a sequence for adding a ribosome binding site to mRNA, a DNA sequence encoding a protein to be expressed, a splicing signal, a terminator sequence for transcription termination, a replication origin sequence, and others. Examples of a preferred animal cell expression vector include: pSV2-X reported by R C. Mulligan et al. [Proc. Natl. Acad. Sci. U.S.A. 78. 2072 (1981)]; and pBP69T (69-6) reported by P. M. Howley et al. [Methods in Emzymology, 101,387, Academic Press (1983)].

Examples of host cells that can be used in production of such peptides include Chinese hamster ovary (CHO) cells, COS-1 cells, COS-7 cells, VERO (ATCC CCL-81) cells, BHK cells, canine kidney-derived MDCK cells, and hamster A-12-664 cells. In addition, examples of such host cells derived from human cells include HeLa cells, WI38 cells, and human 293 cells. Of these cells, CHO cells are extremely common and preferable. Among CHO cells, DHFR-CHO cells are more preferable.

In a genetic engineering process or a peptide production process, microorganisms such as *Escherichia coli* are often used. A host-vector system suitable for each process is preferably used. An adequate vector system can be selected even depending on the aforementioned host cells. A thrombomodulin gene used in a genetic recombination technique has been cloned. Examples of producing thrombomodulin by such a genetic recombination technique have been disclosed. Further, a method for purifying thrombomodulin to obtain a purified product has also been known [JP Patent Publication (Kokai) Nos. 64-6219 (1989) A, 2-255699 (1990) A, 5-213998 (1993) A, 5-310787 (1993) A, and 7-155176 (1995) A; and J. Biol. Chem., 264: 10351-10353 (1989)]. Accordingly, the thrombomodulin used in the present invention can be produced by the methods described in the aforementioned reports, or by methods equivalent thereto. For example, JP Patent Publication (Kokai) No. 64-6219 (1989) A discloses the *Escherichia coli* K-12 strain DH5 (ATCC Accession No. 67283) comprising a plasmid pSV2TMJ2 containing DNA encoding the full-length thrombomodulin. A strain (*Escherichia coli* DH5/pSV2TM J2) (FERM BP-5570), wherein the aforementioned strain has been re-deposited with the former National Institute of Bioscience and Human-Technology (the current National Institute of Advanced Industrial Science and Technology, an Independent Administrative Institution under the Ministry of Economy, Trade and Industry (AIST)), can also be used. The thrombomodulin of the present invention can be prepared by a known genetic engineering technique using the DNA encoding the full-length thrombomodulin as a raw material.

The thrombomodulin used in the present invention may be prepared by a conventionally known method or a method equivalent thereto. For example, the present thrombomodulin can be prepared with reference to the aforementioned method of Yamamoto et al. [JP Patent Publication (Kokai) No. 64-6219 A (1989)] or the method described in JP Patent Publication (Kokai) No. 5-213998 A (1993). That is to say, a human-derived thrombomodulin gene may be subjected to genetic engineering to convert it to DNA encoding the amino acid sequence as shown in SEQ ID NO: 9, for example, and it may be further modified, as necessary. For such modification, a codon encoding the amino acid at position 473 of the amino acid sequence as shown in SEQ ID NO: 9 (particularly, the nucleotide at position 1418) is subjected to site-directed mutagenesis according to the method described in Method in Enzymology, 100: 468 (1983), Academic Press, so as to obtain DNA encoding the amino acid sequence as shown in SEQ ID NO: 11 (which specifically consists of the nucleotide sequence as shown in SEQ ID NO: 12), for example. Using synthetic DNA used for mutation that has the nucleotide sequence as shown in SEQ ID NO: 13, the nucleotide T at position 1418 of SEQ ID NO: 10 may be converted to the nucleotide C, so as to obtain mutated DNA, for example.

The thus prepared DNA is incorporated into, for example, Chinese hamster ovary (CHO) cells to obtain transformant cells. Such cells are then selected, as appropriate. The selected cells are then cultured to obtain a culture solution, and thrombomodulin can be produced from the culture solution by purifying it according to a known method. As stated above, it is preferable that the aforementioned host cells be transfected with the DNA (SEQ ID NO: 10) encoding the amino acid sequence as shown in SEQ ID NO: 9. A method for producing the thrombomodulin used in the present invention is not limited to the aforementioned method. For example, such thrombomodulin may be extracted and purified also from urine, blood, other types of body fluids, etc. Otherwise, it may also be extracted and purified from tissues producing thrombomodulin, a culture solution of such tissues, etc. Further, the thrombomodulin may be further subjected to a cleavage treatment using protease, as necessary.

When the thrombomodulin of the present invention is produced by the aforementioned cell culture method, there may be cases where the N-terminal amino acid becomes diversified as a result of posttranslational modification of protein. For example, the amino acids at positions 17, 18, 19, or 22 of SEQ ID NO: 9 may become the N-terminus in some cases. In addition, there may also be cases where the N-terminal amino acid is modified such that the glutamic acid at position 22 could be converted to pyroglutamic acid. It is preferable that the amino acid at position 17 or 19 become the N-terminus. It is more preferable that the amino acid at position 19 become the N-terminus. Moreover, in another preferred embodiment, the amino acid at position 17 may become the N-terminus. With regard to the aforementioned modification, diversification, etc., the same holds true for SEQ ID NO: 11.

Furthermore, when the thrombomodulin of the present invention is produced using DNA having the nucleotide sequence as shown in SEQ ID NO: 10, the C-terminal amino acid may become diversified, and as a result, a peptide that is shorter than the ordinary peptide by one amino acid residue may be produced in some cases. That is, there may be cases where the amino acid at position 515 becomes the C-terminus, and it is further amidated, so that the C-terminal amino acid can be modified. As a result, a peptide with diversified N-terminal amino acid and C-terminal amino acid, or a mixture thereof, may be produced. The amino acid at position 515 may preferably become the C-terminus. Moreover, in another preferred embodiment, the amino acid at position 516 may become the C-terminus. With regard to the aforementioned modification, diversification, etc., the same holds true for DNA having the nucleotide sequence as shown in SEQ ID NO: 12.

The thrombomodulin obtained by the aforementioned method may be a peptide mixture with diversified N-terminus and C-terminus. A specific example is a mixture of peptides consisting of a sequence consisting of amino acids at positions 19 to 516, at positions 19 to 515, at positions 17 to 516, or at positions 17 to 515 of the amino acid sequence as shown in SEQ ID NO: 9.

Next, isolation and purification of thrombomodulin from the thus obtained culture supernatant or culture product can be carried out a known method [edited by Takeichi Horio, *Tanpakushitsu*/*Koso no Kiso Jikken Ho* (Basic Experimental Methods for Proteins and Enzymes)]. For example, it is preferable to use or adsorption chromatography or ion exchange chromatography which utilizes the interaction between a chromatographic carrier, on which a functional group having a charge opposite to that of thrombomodulin has been immobilized, and thrombomodulin. Another preferred example is affinity chromatography utilizing specific affinity with thrombomodulin. Preferred examples of an adsorbent used herein include thrombin that is a ligand of thrombomodulin and a thrombomodulin antibody. As such an antibody, an antibody of thrombomodulin that has appropriate properties or recognizes appropriate epitopes can be used. For example, those described in JP Patent Publication (Kokoku) No. 5-42920 B (1993), JP Patent Publication (Kokai) Nos. 64-45398 A (1989) and 6-205692 A (1994), etc. can be used. Other examples include gel filtration chromatography and ultrafiltration, which utilize the molecular size of thrombomodulin. Further, other examples include hydrophobic chromatography that utilizes the hydrophobic bond between a chromatographic carrier, on which a hydrophobic group has been immobilized, and the hydrophobic portion of thrombomodulin. Furthermore, hydroxyapatite may be used as a carrier in adsorption chromatography. Such a technique is described in JP Patent Publication (Kokai ) No. 9-110900 A(1997), for example. These means may be used in combination, as appropriate. The degree of purification can be selected depending on intended use and the like. It is desirable to purify thrombomodulin until the results of electrophoresis, and preferably SDS-PAGE, can be obtained in the form of a single band, or until the results of gel filtration HPLC or reverse phase HPLC of the isolated and purified product can be obtained in the form of a single peak. Needless to say, when multiple types of thrombomodulins are used, it is preferable to obtain a band of substantially thrombomodulin only. Thus, it is not necessary to obtain a single band.

A specific example of the purification method applied in the present invention is a purification method using thrombomodulin activity as an indicator. For example, there is applied a purification method [Gomi K. et al., Blood, 75: 1396-1399 (1990)], which comprises: roughly purifying a culture supernatant or a culture product with an ion exchange column Q-Sepharose Fast Flow to recover a fraction having thrombomodulin activity; then mainly purifying it with an affinity column that is a DIP-thrombin-agarose (diisopropylphosphorylthrombin agarose) column to recover a fraction having strong thrombomodulin activity; then concentrating the recovered fraction; and then subjecting the concentrated fraction to gel filtration, so as to obtain a thrombomodulin active fraction as a pure product An example of the thrombomodulin activity used as an indicator is activity of promoting the activation of protein C caused by thrombin. Another preferred purification method will be described below.

A suitable ion exchange resin having good adsorptive condition for thrombomodulin is selected, and purification is then carried out by ion exchange chromatography using it. As a particularly preferred example, a Q-Sepharose Fast Flow that has been equilibrated with a 0.02 M Tris-HCl buffer (pH 7.4) containing 0.18 M NaCl is used. After adequate washing, the resultant is eluted with a 0.02 M Tris-HCl buffer (pH 7.4) containing 0.3 M NaCl, for example, so as to obtain thrombomodulin as a roughly purified product.

Subsequently, for example, a substance having specific affinity with thrombomodulin may be immobilized on a resin, and affinity chromatography may be then carried out Preferred examples include a DIP-thrombin-agarose column and an anti-thrombomodulin monoclonal antibody column. In the case of the DIP-thrombin-agarose column, the column has previously been equilibrated with a 20 mM Tris-HCl buffer (pH7.4) containing 100 mM NaCl and 0.5 mM calcium chloride, and the aforementioned roughly purified product is then charged thereto, followed by adequate washing, for example. Thereafter, the resultant is eluted with a 20 mM Tris-HCl buffer (pH 7.4) containing 1.0 M NaCl and 0.5 mM calcium chloride, for example, so as to obtain thrombomodulin as a purified product. On the other hand, in the case of the anti-thrombomodulin monoclonal antibody column, the following method is applied, for example. An anti-thrombomodulin monoclonal antibody dissolved in a 0.5 M NaCl-containing 0.1 M NaHCO3 buffer (pH 8.3) is allowed to come into contact with Sepharose 4 FF (GE Health Care Biosciences) that has previous been activated by CNBr, so that the anti-thrombomodulin monoclonal antibody is coupled to the Sepharose 4FF. Thereafter, a column filled with a resin wherein the anti-thrombomodulin monoclonal antibody had been coupled to the Sepharose 4FF has previously been equilibrated with a 20 mM phosphate buffer (pH 7.3) containing 0.3 M NaCl, for example. After adequate washing, the resultant is eluted with a 0.3 M NaCl-containing 100 mM glycine-HCl buffer (pH 3.0). The eluant may be neutralized with a suitable buffer, so that it may be obtained as a purified product.

Subsequently, the pH of the purified product is adjusted to pH 3.5, and it is then charged to a cation exchanger, and preferably to SP-Sepharose FF (GE Health Care Biosciences) that is a strong cation exchanger, which has been equilibrated with a 100 mM glycine-HCl buffer (pH 3.5) containing 0.3 M NaCl. It is washed with the same above buffer to obtain a nonadsorption fraction. The obtained fraction is neutralized with a suitable buffer to obtain a highly purified product. Such a product is preferably concentrated by ultrafiltration.

Further, the exchange of a buffer is preferably carried out by gel filtration. For example, the highly purified product concentrated by ultrafiltration is changed to a Sephacryl S-300 column or S-200 column equilibrated with a 20 mM phosphate buffer (pH 7.3) containing 50 mM NaCl, and it is then developed and fractionated using a 20 mM phosphate buffer (pH 7.3) containing 50 mM NaCl. Thereafter, the activity of promoting the activation of protein C caused by thrombin is confirmed, and an active fraction is then recovered, so that a buffer-exchanged highly purified product can be obtained. In order to enhance safety, the thus obtained highly purified product is preferably filtrated using a suitable filter for virus removal such as Planova 15N (Asahi Kasei Medical Co., Ltd.). Thereafter, the product can be concentrated by ultrafiltration to a concentration of interest. Finally, the product is preferably filtrated with an aseptic filtration filter.

The thus obtained thrombomodulin can be processed into a freeze-dried preparation according to an ordinary method. Namely, there is a method, which comprises freezing a solution that contains thrombomodulin and, as necessary, additives, and then drying the frozen product by sublimating water under reduced pressure.

In the present invention, the plasma AT activity may be either AT activity in plasma derived from artery, or AT activity in plasma derived from vein. The plasma AT activity derived from vein is preferable. However, there are also cases where the plasma AT activity derived from artery is preferable. In general, the term "plasma AT activity" is used to mean vein-derived plasma AT activity. The type of a method for measuring AT activity is not particularly limited. For example, such AT activity can be measured by a chromogenic synthetic substrate method of obtaining AT activity based on the residual amount of activated factor X.

Specifically, a blood sample is collected into a blood collecting tube that contains sodium citrate. Thereafter, the blood sample is rapidly centrifuged to recover plasma. Subsequently, heparin is added to the plasma to form an AT-heparin complex, and an excessive amount of activated factor X is then added to the AT-heparin complex. The activated factor X forms an AT-heparin-activated factor X complex depending on the amount of the AT-heparin complex in the sample, so that it is inactivated. After completion of the reaction of forming the aforementioned complex, a substrate solution is added to the reaction solution, and p-nitroaniline released as a result of the reaction is then subjected to colorimetry, so as to measure the activity of the remaining activated factor X. The remaining activated factor X activity reflects AT activity contained in the sample. Thus, the AT activity can be obtained based on a calibration curve that has been produced, separately. An automatic analyzer, BM1650 (manufactured by JEOL Ltd.) can be used in the colorimetry of p-nitroaniline. Further, the calibration curve can be produced using standard substances.

The daily dose of the agent for therapy and/or improvement of DIC comprising the thrombomodulin as an active ingredient according to the present invention differs depending on the age and body weight of patients, the degree of symptoms, an administration route, etc. In general, the upper limit of the amount of thrombomodulin is preferably 5 mg/kg or less, more preferably 2 mg/kg or less, further preferably 1 mg/kg or less, and particularly preferably 0.8 mg/kg or less. The lower limit of the amount of thrombomodulin is preferably 0.005 mg/kg or more, more preferably 0.01 mg/kg or more, further preferably 0.02 mg/kg or more, and particularly preferably 0.05 mg/kg or more.

The agent is applied once or divided over several administrations per day. As administration intervals, the agent may be administered every day. However, it can also be administered preferably once for 2 to 14 days, more preferably once for 3 to 10 days, and further preferably once for 4 to 7 days.

In particular, in the case of intravenous administration, the agent is preferably administered once per day. However, the administration intervals are not limited thereto. On the other hand, in the case of subcutaneous administration, the agent is preferably administered once per day or once per week. It is also possible to adjust such administration intervals depending on the applied dose.

The agent for therapy and/or improvement of DIC comprising the thrombomodulin as an active ingredient according to the present invention can be administered to patients by commonly used administration methods, namely, parenteral methods such as intravenous administration, intramuscular administration, or subcutaneous administration. Of these, intramuscular administration and subcutaneous administration are particularly preferable in that the blood concentration of the agent is maintained for a long period of time and thus that administration intervals can be prolonged. In addition, in another preferred embodiment, intravenous administration is preferably applied. Moreover, oral administration, intrarectal administration, intranasal administration, sublingual administration, and the like are also possible.

In the case of intravenous administration, a method of administering a desired amount of agent at once or by an intravenous drip administration is applied.

Such a method of administering a desired amount of agent at once is preferable in terms of a short administration time. When the agent is administered at once, the time required for administration using an injection syringe generally varies. The time required for administration depends on the amount of liquid to be administered. However, it is preferably 2 minutes or less, more preferably 1 minute or less, and further preferably 30 seconds or less. The lower limit of the time is not particularly limited. It is preferably 1 second or more, more preferably 5 seconds or more, and further preferably 10 seconds or more. The dose applied by this method is not particularly limited, as long as it is within the aforementioned preferred dose.

The intravenous drip administration is preferable in that the concentration of thrombomodulin in blood is easily kept constant The dose applied by the intravenous drip administration is not particularly limited, as long as it is within the aforementioned preferred dose. The upper limit of the dose per day is preferably 1 mg/kg or less, more preferably 0.5 mg/kg or less, further preferably 0.1 mg/kg or less, particularly preferably 0.08 mg/kg or less, and most preferably 0.06 mg/kg or less. The lower limit of the dose per day is preferably 0.005 mg/kg or more, more preferably 0.01 mg/kg or more, further preferably 0.02 mg/kg or more, and particularly preferably 0.04 mg/kg or more.

In the case of the intravenous drip administration, the upper limit of the administration time is preferably 4 hours or less, more preferably 3 hours or less, further preferably 2 hours or less, particularly preferably 1 hour or less, and most preferably 30 minutes or less. The lower limit of the administration time is preferably 10 minutes or more, more preferably 15 minutes or more, and further preferably 20 minutes or more.

When the present agent is processed into a freeze-dried preparation, the preparation may be dissolved in water such as distilled water (or water for injection) or a normal saline solution when it is used, and it may be then administered to patients.

When a liquid preparation is produced from the present agent, an effective amount of thrombomodulin may be mixed with a carrier that can be used as an agent, so as to prepare a liquid preparation. Specifically, thrombomodulin in an amount effective for treating the aforementioned diseases is mixed with a suitable amount of known carrier, so as to produce a preparation suitable for being effectively administered to patients. For example, thickeners such as sucrose, glycerin, methylcellulose or carboxymethylcellulose, pH adjusters such as various types of inorganic salts, and others are added as additives to thrombomodulin, so as to prepare a liquid preparation.

### [Explanation of the sequence listing]

SEQ ID NO: 1 an amino acid sequence encoded by the gene used in production of TME456
SEQ ID NO: 2 a nucleotide sequence encoding the amino acid sequence as shown in SEQ ID NO:1
SEQ ID NO: 3 an amino acid sequence encoded by the gene used in production of TME456M
SEQ ID NO: 4 a nucleotide sequence encoding the amino acid sequence as shown in SEQ ID NO: 3
SEQ ID NO: 5 an amino acid sequence encoded by the gene used in production of TMD12
SEQ ID NO: 6 a nucleotide sequence encoding the amino acid sequence as shown in SEQ ID NO: 5
SEQ ID NO: 7 an amino acid sequence encoded by the gene used in production of TMD12M
SEQ ID NO: 8 a nucleotide sequence encoding the amino acid sequence as shown in SEQ ID NO: 7
SEQ ID NO: 9 an amino acid sequence encoded by the gene used in production of TMD123
SEQ ID NO: 10 a nucleotide sequence encoding the amino acid sequence as shown in SEQ ID NO: 9
SEQ ID NO: 11 an amino acid sequence encoded by the gene used in production of TMD123M
SEQ ID NO: 12 a nucleotide sequence encoding the amino acid sequence as shown in SEQ ID NO: 11
SEQ ID NO: 13 synthetic DNA for mutation, which is used when site-directed mutagenesis is carried out

### EXAMPLES

The present invention will be described in detail in the following examples and test examples. However, these examples are not intended to limit the scope of the present invention.

The thrombomodulin of the present invention used in the following test examples was produced according to the aforementioned method of Yamamoto et al. (the method described in JP Patent Publication (Kokai) No. 64-6219 (1989) A). Production examples of producing the present thrombomodulin will be described below.

### [Production example 1]

### <Obtainment of thrombomodulin>

A highly purified product was obtained by the aforementioned method. Specifically, Chinese hamster ovary (CHO) cells were transfected with DNA (which specifically consists of the nucleotide sequence as shown in SEQ ID NO: 10) encoding the amino acid sequence as shown in SEQ ID NO: 9. Thereafter, an active fraction was recovered from a culture solution of the transformant cells by the aforementioned ordinary purification method using a 20 mM phosphate buffer (pH 7.3) containing 50 mM NaCl, so as to obtain a highly purified product. Thereafter, it was subjected to ultrafiltration to obtain a thrombomodulin solution having a concentration of 11.2 mg/mL (hereinafter abbreviated as TMD123, at times).

### <Preparation of additive solution>

480 g of arginine hydrochloride (manufactured by Ajinomoto Co., Inc.) was weighed out and it was then placed to a 10-L stainless-steel vessel. Thereafter, 5 L of water for injection was added thereto, so that the substance could be dissolved therein. AIM sodium hydroxide solution was further added to the solution, so as to adjust the pH value to pH 7.3.

### <Preparation and addition of agent solution>

The total amount of the aforementioned additive solution was placed to a 20-L stainless-steel vessel, and 2398 mL of the obtained TMD123 solution (corresponding to 26.88 g of soluble thrombomodulin protein; 12% excessive amount added) was added thereto, followed by mixing and stirring. Water for injection was further added to the mixed solution to a total amount of 12 L. The obtained solution was uniformly mixed and stirred. The thus obtained agent solution was subjected to filtration sterilization using a filter having a pore diameter of 0.22 µm (MCGL10S, manufactured by Millipore). Thereafter, 1 ml each of the filtrate was filled into a vial, and the vial was then half-capped with a rubber stopper.

### <Freeze-drying>

A freeze-drying process was carried out in the order of freeze-drying → filling with nitrogen → capping with a rubber stopper → closing the cap by winding it, under the conditions as described below, so as to obtain a TMD123-containing preparation that contained 2 mg of soluble thrombomodulin and 40 mg of arginine hydrochloride in a single vessel.

### <Conditions for freeze-drying>

Preliminary cooling (from room temperature to 15°C over 15 minutes) → Principal cooling (from 15°C to -45°C over 2 hours) → Retention (at -45°C for 2 hours) → Initiation of vacuation (at -45°C for 18 hours) → Temperature rising (from -45°C to 25°C over 20 hours) → Retention (at 25°C for 15 hours) → Temperature rising (from 25°C to 45°C over 1 hour) → Retention (at 45°C for 5 hours) → Room temperature (from 45°C to 25°C over 2 hours) → Recovery of pressure and filling with nitrogen (up to -100 mmHg) → Whole capping → Closing the cap by winding it

### [Production example 2]

Chinese hamster ovary (CHO) cells were transfected with DNA (which specifically consists of the nucleotide sequence as shown in SEQ ID NO: 12) encoding the amino acid sequence as shown in SEQ ID NO: 11. Thereafter, a thrombomodulin solution (hereinafter abbreviated as TMD123M, at times) was obtained from a culture solution of the transformant cells by the aforementioned ordinary purification method. Thereafter, a freeze-dried preparation of TMD123M was obtained by the same method as that described above.

### [Production example 3]

Chinese hamster ovary (CHO) cells were transfected with DNA (which specifically consists of the nucleotide sequence as shown in SEQ ID NO: 2) encoding the amino acid sequence as shown in SEQ ID NO: 1. Thereafter, thrombomodulin (hereinafter abbreviated as TME456, at times) was obtained from a culture solution of the transformant cells by the aforementioned ordinary purification method. Thereafter, a freeze-dried preparation of TME456 was obtained by the same method as that described above.

### [Production example 4]

Chinese hamster ovary (CHO) cells were transfected with DNA (which specifically consists of the nucleotide sequence as shown in SEQ ID NO: 4) encoding the amino acid sequence as shown in SEQ ID NO: 3. Thereafter, thrombomodulin (hereinafter abbreviated as TME456M, at times) was obtained from a culture solution of the transformant cells by the aforementioned ordinary purification method. Thereafter, a freeze-dried preparation of TME456M was obtained by the same method as that described above.

### [Production example 5]

Chinese hamster ovary (CHO) cells were transfected with DNA (which specifically consists of the nucleotide sequence as shown in SEQ ID NO: 6) encoding the amino acid sequence as shown in SEQ ID NO: 5. Thereafter, thrombomodulin (hereinafter abbreviated as TMD12, at times) was obtained from a culture solution of the transformant cells by the aforementioned ordinary purification method. Thereafter, a freeze-dried preparation of TMD12 was obtained by the same method as that described above.

### [Production example 6]

Chinese hamster ovary (CHO) cells were transfected with DNA (which specifically consists of the nucleotide sequence as shown in SEQ ID NO: 8) encoding the amino acid sequence as shown in SEQ ID NO: 7. Thereafter, thrombomodulin (hereinafter abbreviated as TMD12M, at times) was obtained from a culture solution of the transformant cells by the aforementioned ordinary purification method. Thereafter, a freeze-dried preparation of TMD12M was obtained by the same method as that described above.

### [Test example 1]

The agent for therapy and/or improvement of DIC of the present invention prepared by a method described below was administered to DIC patients, as described below, who had been diagnosed as DIC based on the DIC diagnostic criteria of the Health and Welfare Ministry, whose symptoms of DIC had been induced from hemopoietic malignancy or infectious disease. The survival rate of the patients on the 28^{th} day after initiation of the administration was examined.

It is to be noted that an AT preparation was not administered during the administration of the agent for therapy and/or improvement of DIC of the present invention.

### <Formulation example 1>

A normal saline solution was added to a TMD123 freeze-dried preparation obtained according to Production example 1 to prepare a 0.5 mg/ml test solution.

### <Measurement of AT activity>

On the day of the administration of the agent for therapy and/or improvement of DIC of the present invention, 4.5 ml of blood sample was collected from a target patient before administration of the agent, and it was then placed into a blood collecting tube that contained 0.5 ml of 3.8% sodium citrate. Thereafter, they were mixed with each other by turning the tube upside down, and the mixture was then centrifuged. Thus, plasma was recovered and was then cryogenically preserved. Subsequently, heparin was added to the plasma to form an AT-heparin complex. An excessive amount of factor Xa was further added to the AT-heparin complex. After the reaction of forming the complex, a substrate solution (Testzyme S ATIII (manufactured by Chromogenix)) was added thereto, and p-nitroaniline released as a result of the reaction was then subjected to colorimetry by absorbance determination at 410 nm, so as to measure the remaining factor Xa activity (instruction for use included with Testzyme S ATIII). Since the remaining factor Xa activity reflects AT activity contained in the sample, the AT activity was obtained using a calibration curve that had been produced, separately. An automatic analyzer BM1650 (manufactured by JEOL Ltd.) was used in the colorimetry of p-nitroaniline. In addition, a normal plasma preparation "Daiichi" (Daiichi Kagaku Co., Ltd.) was used as normal plasma to obtain such AT activity.

### <Dose and administration method>

As to the target DIC patients, the test solution prepared in the aforementioned formulation example was added at a rate of 0.12 ml/kg (corresponding to 0.06 mg/kg) to a normal saline solution to a total amount of 100 ml. The thus prepared normal saline solution was administered once per day over 30 minutes via intravenous drip administration. Such administration was continued for 6 days.

### <Results>

The test solution was administered by the aforementioned administration method. Thereafter, the outcome was examined on 28^{th} day after initiation of the administration. As a result, the survival rate of a patient group having an AT activity between 50% or more and less than 70% was found to be 64.3%, whereas the survival rate of a patient group having an AT activity of 70% or more was found to be 80%. On the other hand, the survival rate of a patient group having an AT activity of less than 50% was found to be 92.3%, including only 1 fatal case. Thus, the patient group having an AT activity of less than 50% exhibited a survival rate superior to those of the two above patient groups. These results are shown in Table 1.

Moreover, among others, patients having an AT activity of 40% or less (5 cases) exhibited a survival rate of 100%.

**[Table 1]**

| AT activity (%) | Survived | | Died | |
|---|---|---|---|---|
| | Number of cases | % | Number of cases | % |
| < 50 | 12 | 92.3 | 1 | 7.7 |
| 50 ≦ and < 70 | 18 | 64.3 | 10 | 35.7 |
| 70 ≦ | 60 | 80.0 | 15 | 20.0 |

### [Test example 2]

A rabbit anti-rat AT antibody (20 mg/kg) is administered into the caudal vein of each male Sprague-Dawley rat (body weight: 180 to 240 g), so as to decrease a rat plasma AT activity to less than 60%. One hour after administration of the antibody, the rat was subjected to pentobarbital anesthesia, and a tissue factor (rabbit-derived thromboplastin: 90 mg/kg) is continuously administered into the caudal vein of the rat over 1 hour. Immediately before administration of the tissue factor, 0.1 to 10 mg/kg thrombomodulins in various production examples of the present invention are rapidly administered to the caudal vein on the opposite side. After completion of the administration of the tissue factor, blood was collected from the abdominal aorta of each rat. Thereafter, the number of thrombocytes, a fibrinogen value, or AT activity is measured to examine the effect of each thrombomodulin for therapy and/or improvement of DIC, thereby confirming the effect of the present invention.

### INDUSTRIAL APPLICABILITY

Using the thrombomodulin-containing agent of the present invention, the symptoms of DIC patients whose plasma AT activity is 50% or less can be effectively treated and/or improved, preferably without combining with AT.

## Claims

1. An agent for therapy and/or improvement of disseminated intravascular coagulation comprising thrombomodulin as an active ingredient, which is administered to patients suffering from such disseminated intravascular coagulation who have a plasma antithrombin activity of less than 50%.

2. The agent for therapy and/or improvement of disseminated intravascular coagulation according to claim 1, wherein said thrombomodulin is soluble thrombomodulin.

3. The agent for therapy and/or improvement of disseminated intravascular coagulation according to claim 1 or 2, wherein said thrombomodulin is a peptide obtained from transformant cells prepared by transfecting host cells with DNA encoding the amino acid sequence as shown in SEQ ID NO: 9 or 11.

4. The agent for therapy and/or improvement of disseminated intravascular coagulation according to any one of claims 1 to 3, wherein said thrombomodulin is a peptide having a sequence consisting of amino acids at positions 19 to 516 of the amino acid sequence as shown in SEQ ID NO: 9 or 11, or a peptide having an amino acid sequence comprising a substitution, deletion or addition of one or multiple amino acids with respect to the amino acid sequence of said peptide and having thrombomodulin activity.

5. The agent for therapy and/or improvement of disseminated intravascular coagulation according to any one of claims 1 to 4, wherein the patients suffering from disseminated intravascular coagulation have a plasma antithrombin activity of 40% or less.

6. The agent for therapy and/or improvement of disseminated intravascular coagulation according to any one of claims 1 to 5, which is not administered in combination with antithrombin.

7. An agent for reducing the likelihood of death in patients suffering from disseminated intravascular coagulation, which comprises thrombomodulin as an active ingredient and which is administered to the patients suffering from disseminated intravascular coagulation who have a plasma antithrombin activity of less than 50%.

8. A method for selecting patients suffering from disseminated intravascular coagulation, to whom thrombomodulin is to be administered, which comprises measuring the plasma antithrombin activity of patients suffering from disseminated intravascular coagulation, selecting patients suffering from disseminated intravascular coagulation whose antithrombin activity is less than 50%, and determining that they are patients suffering from disseminated intravascular coagulation, to whom thrombomodulin is to be administered.

9. A method for treating and/or improving disseminated intravascular coagulation, which comprises administering thrombomodulin to patients suffering from disseminated intravascular coagulation whose plasma antithrombin activity is less than 50%.

10. An agent for therapy and/or improvement of disseminated intravascular coagulation comprising thrombomodulin as an active ingredient, which is administered to patients suffering from disseminated intravascular coagulation after their plasma antithrombin activity has been measured.
